# NEW EUROPEAN PATENT SPECIFICATION

(11) **EP 1 355 623 B2**
(45) Date of publication and mention of the opposition decision: **21.12.2011**
(45) Mention of the grant of the patent: 24.01.2007
(21) Application number: 01910406.6
(22) Date of filing: 01.02.2001
(51) Int. Cl.: A61K 8/23, A61Q 5/12

(54) **ANHYDROUS COSMETIC COMPOSITIONS**
WASSERFREIE KOSMETISCHE ZUSAMMENSETZUNGEN
COMPOSITIONS COSMETIQUES ANHYDRES

(43) Date of publication of application: 29.10.2003
(73) Proprietor: THE PROCTER & GAMBLE COMPANY, Cincinnati, OH 45202 (US)
(72) Inventor: UCHIDA, Mikio, Ashiyo, Hyogo 659-0016 (JP); MITSUMATSU, Arata, Hyogo 662-0018 (JP); KAWAUCHI, Akihiko, Hyogo 662-0934 (JP); AZUMA, Misa, Osaka 581-0843 (JP)
(74) Representative: Simpson, Kirsty Mairi
(86) International application number: PCT/US2001/003425
(87) International publication number: WO 2002/060408

(56) References cited:
- EP-A- 0 027 730
- EP-A- 0 586 929
- WO-A-00/38621
- WO-A-01/08654
- WO-A-01/12150
- WO-A1-00/09082
- WO-A1-00/38621
- DE-A1- 19 624 870
- GB-A- 1 042 529
- JP-A- 57 099 514
- JP-T1- 57 099 514
- US-A- 3 250 680
- ICI AMERICAS INC.: 'ICI Surfactants', 03 March 1994 article 'Arlatone 289(Technical Bulletin)'
- J.A.WENNINGER ET AL: 'International Cosmetic Ingredient Dictionary', vol. 1, 1993, THE COSMETIC, TOILETRY, AND FRAGRANCE ASSOCIATION, WASHINGTON,D.C., ISBN 1-882621-06-9 article 'PEG-54 Hydrogenated Castor Oil , Trade name: Arlatone 289 (ICI)'
- HENKEL AG & CO. KGAA: 'Cospha: product information Cutina EGMS', September 1996
- ICI AMERICAS INC.: 'ICI Surfactants', 31 August 1993 article 'Atlas G-1821(Technical Bulletin)'
- RHODIA: 'Rhodasurf ON-870', March 2000 article 'Product Data Sheet'
- 'Lanette®O', April 1999 article PRODUCT DATA SHEET
- ATLAS CHEMIE: 'Brij®', 30 April 1990 article PRODUCT INFORMATION
- UNIQEMA: 'Data Sheet for Brij 72', 07 March 2000
- ICI AMERICAS INC.: 'ICI Surfactants', 06 August 1993 article "BRIJ" 721
- H.P.FIEDLER: 'Lexikon der Hilfsstoffe für Pharmazie, Kosmetik und Angrenzende Gebiete', 1971, EDITIO CANTOR KG, AULENDORF, ISBN 3 87193 002 4 pages 467 - 468
- 'Encyclopedia of Excipients', INTERNET article ' Arlatone 289'

## Description

### TECHNICAL FIELD

The present invention relates to an anhydrous hair care and/or skin care cosmetic composition which warms by mixing with water.

### BACKGROUND

A variety of cosmetic products such as hair care products and skin care products have been used to the hair and/or skin. With respect to hair care products, for example, hair shampoo products are used for cleansing the hair by removing excess soil and sebum; hair conditioning products are used for providing various conditioning benefits such as moisturized feel, softness, and static control to the hair; hair styling products are used for setting hair style and/or maintaining hair style; hair color products are used for changing hair color and/or maintaining hair color; and hair growth products are used for encouraging hair growth.

The efficacy of cosmetic products such as hair care products and skin care products are changed by various factors, for example, amount of products applied, how long products are applied on the hair, temperatures of products, the way of applying products to the hair, and so on. Thus, it may not be easy to obtain expected efficacy from cosmetic products such as hair care products and skin care products.

A variety of approaches have been developed to obtain expected efficacy from hair care products. For example, inorganic salts and/or oxides which generate a heat by mixing with water are contained in anhydrous cosmetic compositions, being expected to improve penetration and deposition of conditioning components to skin and/or hair by its heat generating (described in Japanese Patent Laid-open H11-228332). However, it has been found that; inorganic heat generating agents such as inorganic salts and/or oxides sometimes generate greater heat than expected, thus the compositions warm up to a higher temperature than expected. It has been also found that; inorganic heat generating agents such as inorganic salts and/or oxides generate a heat over only a short period of time, thus, it is difficult to obtain prolonged warming from the compositions.

Japanese Patent Laid-open No. H11-228333 discloses a heat generating cosmetics comprising a heat generating agent coated by a silicone type coating agent, for providing mild heat changing and prolonged warming. However, coated heat generating agents are generally more expensive than non-coated heat generating agents.

Based on the foregoing, there remains a desire for obtaining enhanced efficacy from cosmetic products such as hair care products and skin care products, i.e., a desire for obtaining improved benefits from cosmetic products. With respect to hair care products, for example, there remains a desire for obtaining improved cleansing benefits from hair shampoo products, and obtaining improved hair conditioning benefits such as moisturized feel, softness, and static control from hair conditioning products, while controlling the temperature to which the composition warms up. There also remains a desire for obtaining prolonged warming from cosmetic products such as hair care products and skin care products. There also remains a desire for providing such cosmetic products which can provide above benefits with minimum cost increase.

None of the existing art provides all of the advantages and benefits of the present invention.

### SUMMARY

The present invention is directed to an anhydrous hair care and/or skin care cosmetic composition comprising:
(a) a heat generating agent which generates a heat by mixing with water;
(b) a phase changing agent; and
(c) an inert carrier;
wherein the phase changing agent has a melting point of from about 30°C to about 70°C and are dispersed in the inert carrier and wherein the heat generating agent is an anhydrous inorganic salt selected from the group consisting of sodium sulfate, calcium sulfate, magnesium sulfate aluminum sulfate, calcium chloride, magnesium chloride, calcium oxide, and mixtures thereof.

These and other features, aspects, and advantages of the present invention will become evident to those skilled in the art from a reading of the present disclosure.

The present invention provides anhydrous hair care and/or skin care cosmetic compositions which warm by mixing with water. The compositions of the present invention can provide enhanced efficacy while controlling the temperature which the composition warms to. The compositions of the present invention can also provide prolonged warming from cosmetic products. It is believed that; warming cosmetic compositions such as hair care compositions and skin care compositions can provide enhanced efficacy, i.e., can provide improved benefits. With respect to hair care compositions, for example, it is believed that; warming hair shampoo compositions can provide improved cleansing benefits, warming hair styling compositions can provide improved styling benefits, warming hair conditioning compositions can provide improved hair conditioning benefits due to improved penetration of ingredients, and warming hair color compositions and warming hair growth compositions can also provide improved benefits. With respect to skin care compositions, for example, it is believed that; warming body shampoo compositions can provide improved cleansing benefits, warming face cleansing compositions can provide improved cleansing benefits, warming skin conditioning compositions can provide improved conditioning benefits, and warming shaving compositions can provide improved shaving benefits. It is also believed that; the phase changing agent having a certain melting point of the present invention can absorb a heat from the heat generating agent by changing its phase from solid to liquid, and then, release the heat slowly by changing its phase from liquid to solid. Thus, it is believed that; the phase changing agent can prevent the compositions from warming up to a higher temperature than expected, and provide prolonged warming from the compositions, without using coated heat generating agents.

### DETAILED DESCRIPTION

While the specification concludes with claims particularly pointing out and distinctly claiming the invention, it is believed the present invention will be better understood from the following description.

All percentages are by weight of the total composition unless otherwise indicated. All ratios are weight ratios unless otherwise indicated. All percentages, ratios, and levels of ingredients referred to herein are based on the actual amount of the ingredient, and do not include solvents, fillers, or other materials with which the ingredient may be combined as commercially available products, unless otherwise indicated.

As used herein, "comprising" means that other steps and other ingredients which do not affect the end result can be added. This term encompasses the terms "consisting of" and "consisting essentially of".

All cited references are incorporated herein by reference in their entireties. Citation of any reference is not an admission regarding any determination as to its availability as prior art to the claimed invention.

### ANHYDROUS COSMETIC COMPOSITIONS

The anhydrous cosmetic compositions of the present invention warm by a heat from heat generating agents when mixing with water. As used in the present invention, "anhydrous" means that the compositions contain 5% or less of water. The anhydrous compositions of the present invention contain, preferably 3% or less, more preferably 1% or less, still more preferably substantially free of water. The anhydrous cosmetic compositions warm to a temperature of, preferably from about 30°C to about 80°C, more preferably from about 30°C to about 60°C, still more preferably from about 35 °C to about 45 °C, on hair and/or skin. This temperature can be adjusted by, for example, choosing the heat generating agents, the amount of the heat generating agent, and additional agents which can control the heat generating reaction. The anhydrous cosmetic compositions keep the above temperature for, preferably about 30 sec. or more, more preferably about 1 min. or more, still more preferably about 3 min. or more.

Various anhydrous hair care compositions and anhydrous skin care compositions can be used in the present invention. The anhydrous hair care compositions useful herein include, for example, anhydrous hair shampoo compositions, anhydrous hair styling compositions, anhydrous hair conditioning compositions, anhydrous hair color compositions, anhydrous hair growth compositions, and mixtures thereof. The anhydrous skin care compositions useful herein include, for example, anhydrous body shampoo compositions, anhydrous face cleansing compositions, anhydrous skin conditioning compositions, anhydrous shaving compositions, and mixtures thereof.

It is believed that; warming cosmetic hair care compositions and skin care compositions can provide enhanced efficacy, i.e., can provide improved benefits. With respect to hair care compositions, for example, it is believed that; warming hair shampoo compositions can provide improved cleansing benefits, warming hair styling compositions can provide improved styling benefits, warming hair conditioning compositions can provide improved hair conditioning benefits due to improved penetration of ingredients, warming hair color compositions and warming hair growth compositions can also provide improved benefits. With respect to skin care compositions, for example, it is believed that; warming body shampoo compositions can provide improved cleansing benefits, warming face cleansing compositions can provide improved cleansing benefits, warming skin conditioning compositions can provide improved conditioning benefits, and warming shaving compositions can provide improved shaving benefits.

The anhydrous cosmetic compositions of the present invention can be in the form of rinse-off products or leave-on products, can be transparent or opaque, and can be formulated in a wide variety of product forms, including but not limited to lotions, creams, gels, emulsions, mousses, and sprays.

The anhydrous cosmetic compositions of the present invention can be mixed with water and applied to the hair and/or skin by any conventional method well known in the art. For example, the anhydrous compositions can be applied to hair and/or skin after mixing with water on hands and/or in a certain vessel. The anhydrous compositions can be applied to wet hair and/or wet skin to mix with water remaining on the hair and/or skin. The anhydrous compositions can be applied to wet and/or dry hair and/or skin to mix with water when rinsed-off.

### HEAT GENERATING AGENT

The anhydrous cosmetic compositions of the present invention comprise a heat generating agent which generates a heat by mixing with water.

The amount and type of heat generating agents are selected so that the composition is brought to a temperature higher than the melting point of the phase changing agents when mixed with water. In order to generate such a heat, the heat generating agents should be able to heat a composition excluding the phase changing agents to a temperature of preferably from about 35°C to about 80°C, more preferably from about 40°C to about 60°C, still more preferably from about 40 °C to about 50 °C.

The heat generating agent is an anhydrous inorganic salt such as sodium sulfate (Na₂SO₄), calcium sulfate (CaSO₄), magnesium sulfate (MgSO₄), aluminum sulfate (Al(SO₄)₃), calcium chloride (CaCl₂), magnesium chloride(MgCl₂), calcium oxide (CaO), and mixtures thereof, in view of their effective heat generation, mildness to hair and/or skin, and easy handling. More preferred is anhydrous magnesium sulfate (MgSO₄).

The heat generating agent is included in the compositions at a level by weight of, preferably from about 5% to about 60%, more preferably from about 15% to about 50%, still more preferably from about 25% to about 45%.

### PHASE CHANGING AGENTS

The anhydrous cosmetic compositions of the present invention comprise a phase changing agent dispersed in an inert carrier. It is also believed that; the phase changing agent having a certain melting point of the present invention can absorb a heat from the heat generating agent by changing its phase from solid to liquid, and then, release the heat slowly by changing its phase from liquid to solid. Thus, it is believed that; the phase changing agent can prevent the compositions from warming up to a higher temperature than expected, and provide prolonged warming from the compositions, without using coated heat generating agents.

The phase-changing agents of the present invention have a melting point of from about 30°C to about 70°C, preferably from about 30 °C to about 60 °C, more preferably from about 35 °C to about 50 °C. This melting point can be that of one kind of material. The melting point can also be a mixture of 2 or more kinds of materials, when 2 or more materials are miscible with each other. In this case, each material does not necessarily have a melting point of from about 30°C to about 70°C, however, mixtures thereof have a melting point of from about 30°C to about 70°C.

The phase-changing agents useful herein include, for example, amidoamines; fatty compounds such as fatty alcohols, fatty acids, fatty alcohol derivatives, fatty acid derivatives, and mixtures thereof; hydrocarbons such as solid paraffin; and mixtures thereof. Fatty compound useful herein are disclosed below under the title "HIGH MELTING POINT FATTY COMPOUND". Amidoamines useful herein are disclosed below under the title "AMIDOAMINE". Preferred phase changing agent are; fatty compounds such as fatty alcohols, fatty acids, fatty alcohol derivatives, fatty acid derivatives, and mixtures thereof; amidoamines; and mixtures thereof, in view of providing conditioning benefit. More preferred are fatty alcohols selected from the group consisting of cetyl alcohol (melting point = 46-55 °C), stearyl alcohol (melting point = 54-61 °C), and mixtures thereof, still more preferred are mixtures of cetyl alcohol and stearyl alcohol having a weight ratio of cetyl alcohol to stearyl alcohol of from about 10 : 90 to about 99 : 1 (melting point = from about 48 °C to about 58 °C)

In order to function as a phase changing agent, materials are dispersed, but not dissolved in an inert carrier. Materials completely dissolved in an inert carrier, even if they have a melting point of from about 30°C to about 70°C, can not function as the phase changing agents of the present invention. For example, Japanese Patent Laid-open No. H11-228332 discloses a composition containing 5wt% of myristyl myristate having a melting point of from 41 to 43 °C and 65wt% of octyl stearate carrier (in Example 9), however, 5wt% of myristyl myristate is completely dissolved in 65wt% of octyl stearate carrier, thus, it can not function as the phase changing agent of the present invention.

In the present invention, in order to make phase changing agents dispersed in an inert carrier, materials which are insoluble in the inert carrier are used, or materials are contained at a higher level than its saturation point in the inert carrier. Materials having a lower solubility to an inert carrier are preferably used. The solubility depends on each combination of phase changing agents and inert carriers. For example, preferable combinations of phase changing agents and inert carriers include; the combination of high melting point fatty compound as phase changing agent, and polyethylene glycol as an inert carrier; the combination of high melting point fatty compound as phase changing agent, and glycerin as an inert carrier; the combination of high melting point fatty compound as phase changing agent, and low melting point ester oils as an inert carrier; the combination of high melting point fatty compound as phase changing agent, and liquid paraffin as an inert carrier; the combination of hydrocarbons such as solid paraffin as phase changing agent, and polyethylene glycol as an inert carrier. More preferred are; the combination of fatty alcohols selected from the group consisting of cetyl alcohol, stearyl alcohol, and mixtures thereof as phase changing agents, and polyethylene glycol as an inert carrier; the combination of fatty alcohols selected from the group consisting of cetyl alcohol, stearyl alcohol, and mixtures thereof as phase changing agents, and glycerin as an inert carrier; the combination of fatty alcohols selected from the group consisting of cetyl alcohol, stearyl alcohol, and mixtures thereof as phase changing agents, and pentaerythritol ester oils as an inert carrier.

The phase changing agent can be included in the compositions at a level by weight of, preferably from about 0.2% to about 20%, more preferably from about 0.5% to about 15% still more preferably from about 1% to about 10%. INERT CARRIER

The anhydrous cosmetic composition of the present invention comprises an inert carrier. The inert carriers useful herein are liquid carriers and include; for example, liquid polyhydric alcohols such as polyethylene glycol, polypropylene glycol, 1,2-propane diol or propylene glycol, 1,3-propane diol, hexylene glycol, glycerin, diethylene glycol, dipropylene glycol, 1,2-butylene glycol, 1,4-butylene glycol; liquid paraffin; mineral oil; vegetable oil; low melting point oil such as pentaerythritol ester oils, trimethylol ester oils, poly α-olefin oils, citrate ester oils, glyceryl ester oils, and mixtures thereof; and mixtures thereof. The liquid polyhydric alcohols such as polyethylene glycol can also be used as "ADDITIONAL HEAT GENERATING AGENTS" described below. The low melting point oil useful herein are described below as a conditioning agent under the title "LOW MELTING POINT OIL". Preferred are polyethylene glycol, polypropylene glycol, glycerin, liquid paraffin, mineral oil, vegetable oil, low melting point ester oils, and mixtures thereof in view of physical properties such as viscosity and fluidity. More preferred is polyethylene glycol in view of its ability to generate a heat by mixing with water and physical properties such as viscosity and fluidity.

The polyethylene glycols useful herein are those having the formula:

H(OCH₂CH₂)ₙ -OH

wherein n has an average value of from 4 to 12.

The polyethylene glycol described above is also known as a polyethylene oxide, and polyoxyethylene. Polyethylene glycols useful herein that are especially preferred are PEG-200 wherein n has an average value of about 4. Commercially available preferred polyethylene glycol includes, for example, PEG-200 having trade name Carbowax PEG-200 available from Union Carbide).

The inert carrier is included in the compositions at a level by weight of, preferably from about 10% to about 90%, more preferably from about 25% to about 90%, still more preferably from about 30% to about 85%.

### POLYOXYALKYLENE DERIVATIVES

The anhydrous cosmetic composition of the present invention may contain polyoxyalkylene derivatives. The polyoxyalkylene derivatives are preferably used in the present invention, when the heat generating agent is an inorganic heat generating agent, in order to prevent the agglomeration of inorganic heat generating agents, which agglomeration causes gritty feel to the skin and /or hair.

The polyoxyalkylene derivatives useful herein are preferably water soluble polyoxyalkylene derivatives.

The polyoxyalkylene derivatives useful herein include, for example, polyoxyethylene/polyoxypropylene copolymer, polyoxyethylene alkyl ether, polyoxypropylene alkyl ether, polyoxyethylene alkyl ether ester, polyoxypropylene alkyl ether ester, polyoxyethylene glyceryl ester, polyoxypropylene glyceryl ester, and mixtures thereof.

Preferred polyoxyethylene alkyl ethers are, for example, those of the formula RO(CH₂CH₂O)ₙH, wherein n is from 1 to about 200, preferably from about 20 to about 100, and R is an alkyl having from about 8 to about 22 carbon atoms.

Preferred polyoxyethylene glyceryl esters include, for example, following (i) and (ii).
(i) PEG-modified glycerides having the structure: 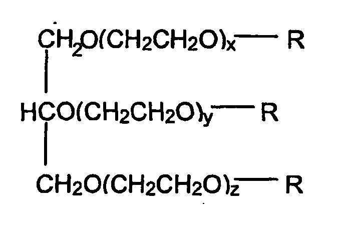 wherein one or more of the R groups is selected from saturated or unsaturated fatty acid moieties derived from animal or vegetable oils such as palmitic acid, lauric acid, oleic acid or linoleic acid wherein the fatty acid moieties have a carbon length chain of from 12 and 22, any other R groups are hydrogen, x, y, z are independently zero or more, the average sum of x+y+z (the degree of ethoxylation) is equal to from about 10 to about 45. Preferably, the PEG-modified glycerides have an HLB value of about 20 or less, more preferably about 15 or less, still preferably about 11 or less. Preferably, the PEG-modified glycerides have from 2 to 3 fatty acid R groups, more preferred have 3 fatty acid R groups (PEG-modified triglycerides). Preferably, the average sum of x+y+z (the degree of ethoxylation) is equal to from about 20 to 30, more preferred is an average sum of 25. Most preferred are PEG-substituted triglycerides having 3 oleic acid R groups, wherein the average degree of ethoxylation is about 25 (PEG-25 glyceryl trioleate). Preferred commercially available PEG-modified triglycerides include Tagat TO ®, Tegosoft GC, Tagat BL 276®, (all three manufactured by Goldschmidt Chemical Corporation) and Crovol A-40, Crovol M-40 (manufactured by Croda Corporation). Other preferred commercially available PEG-modified triglycerides include Tagat S ® and Tagat S 2 ® (manufactured by Goldschmidt Chemical Corporation).
(ii) PEG-modified glyceryl fatty acid esters having the structure: 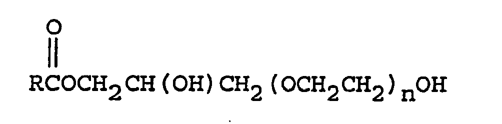 wherein n, the degree of ethoxylation, is from about 4 to about 200, preferably from about 5 to about 150, more preferably from about 20 to about 120, and wherein R comprises an aliphatic radical having from about 5 to about 25 carbon atoms, preferably from about 7 to about 20 carbon atoms. Suitable polyethylene glycol derivatives of glycerides can be polyethylene glycol derivatives of hydrogenated castor oil. For example, PEG-20 hydrogenated castor oil, PEG-30 hydrogenated castor oil, PEG-40 hydrogenated castor oil, PEG-45 hydrogenated castor oil, PEG-50 hydrogenated castor oil, PEG-54 hydrogenated castor oil, PEG-55 hydrogenated castor oil, PEG-60 hydrogenated castor oil, PEG-80 hydrogenated castor oil, and PEG-100 hydrogenated castor oil. Preferred for use in the compositions herein is PEG-60 hydrogenated castor oil. Other suitable polyethylene glycol derivatives of glycerides can be polyethylene glycol derivatives of stearic acid. For example, PEG-30 stearate, PEG-40 stearate, PEG-50 stearate, PEG-75 stearate, PEG-90 stearate, PEG-100 stearate, PEG-120 stearate, and PEG-150 stearate. Preferred for use in the compositions herein is PEG-100 stearate.

Preferred polyoxyethylene/polyoxypropylene copolymers include, for example, polyoxyethylene/polyoxypropylene random copolymer and polyoxyethylene/polyoxypropylene block copolymer.

Among these polyoxyalkylene derivatives, polyoxyethylene/polyoxypropylene copolymers including polyoxyethylene/polyoxypropylene random copolymer and polyoxyethylene/polyoxypropylene block copolymer are preferably used in the composition of the present invention in view of their suspending benefit. More preferred is polyoxyethylene/polyoxypropylene block copolymer, still more preferred is polyoxyethylene/polyoxypropylene block copolymer having a weight ratio of polyoxyethylene to polyoxypropylene of from about 5:10 to about 8:10, even more preferred is the block copolymer having the ratio of 8:10.

Commercially available polyoxyalkylene derivatives useful herein include: polyoxyethylene/polyoxypropylene block copolymer: having CTFA name Poloxamer 338, available from BASF under trade name Pluronic F-108, and also available from Sanyo Chemical under trade name Newpol PE-108; and having CTFA name Poloxamer 288, available from BASF under trade name Pluronic F-98, and also available from Sanyo Chemical under trade name Newpol PE-98.

The polyoxyalkylene derivative can be included in the compositions at a level by weight of, preferably from about 0.1 % to about 10%, more preferably from about 0.5% to about 10%, still more preferably from about 1% to about 5%.

### REACTION CONTROL AGENTS

The anhydrous cosmetic compositions of the present invention preferably contain reaction control agents which can control the heat generating reaction of the heat generating agent. The reaction control agents may slow down the reaction, or accelerate the reaction. The reaction control agents may also control the temperature to which the cosmetic composition warms up.

Acids can be used as reaction control agents for accelerating the reaction of the inorganic heat generating agents. The acid useful herein includes, for example, citric acid, sodium diphosphate, potassium diphophate. ℓ-glutamic acid, lactic acid, hydrochloric acid, malic acid, succinic acid, acetic acid, fumaric acid, ℓ-glutamic acid hydrochloride, tartaric acid, and mixtures thereof; preferably ℓ-glutamic acid, lactic acid, hydrochloric acid, and mixtures thereof. Among the above acids, citric acid is preferably used herein. Some acids can also be used together with amidoamines for providing conditioning benefits as described below. The acid can be contained at a level such that the mole ratio of the inorganic heat generating agent to acid is from about 1:0.1 to about 1:10, preferably from about 1:0.5 to about 1:5.

Water absorbing polymer can be used as reaction control agents for slowing down the reaction of the inorganic heat generating agent. The water absorbing polymer useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers such as Hydroxyethylcellulose and Hydroxypropyl cellulose, polyvinylpyrrolidone, polyvinyl alcohol, guar gum, other gums, starch-based polymers, alginic acid-based polymers, acrylate polymers, polyalkylene glycols having a molecular weight of more than about 1000, and mixtures thereof. These water absorbing polymers can also be used as the "VISCOSITY MODIFYING AGENT" described below. Among the above water absorbing polymers, preferred are cellulose derivatives and modified cellulose polymers, and more preferred is Hydroxyethylcellulose. The water absorbing polymers can be included in the compositions at a level by weight of, preferably from about 0.2% to about 20%, more preferably from about 0.5% to about 15%, still more preferably from about 1% to about 10%.

### HEAT RESERVING MATERIALS

The anhydrous cosmetic compositions of the present invention may contain heat reserving materials which can reserve a heat. The heat reserving material can be used for prolonging heating, and may be used for slowing down the warming speed, and may control the temperature to which the cosmetic composition warms up.

The heat reserving materials include, for example, silica gel, carboxymethyl cellulose gel, and mixtures thereof.

The heat reserving material can be included in the compositions at a level by weight of, preferably from about 0.2% to about 20%, more preferably from about 0.5% to about 15% still more preferably from about 1% to about 10%. VISCOSITY MODIFYING AGENT

The anhydrous cosmetic composition of the present invention may contain a viscosity modifying agent. The viscosity modifying agent useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers, polyvinylpyrrolidone, polyvinyl alcohol, guar gum, other gums, starch-based polymers, alginic acid-based polymers, acrylate polymers, polyalkylene glycols having a molecular weight of more than about 1000, inorganic water soluble material such as bentonite, aluminum magnesium silicate, laponite, hectorite, and anhydrous silicic acid, and mixtures thereof. The polymers described herein can also be used as the "VISCOSITY MODIFYING AGENT" described above. Some polyalkylene glycols described herein can also be used as hair conditioning agents described below under the title "POLYPROPYLENE GLYCOL" and "POLYETHYLENE GLYCOL".

The viscosity modifying agent can be included in the compositions at a level by weight of, preferably from about 0.01% to about 5%, more preferably from about 0.05% to about 3% still more preferably from about 0.1% to about 3%.

### ADDITIONAL HEAT GENERATING AGENTS

The anhydrous cosmetic compositions of the present invention may contain additional heat generating agents in addition to the heat generating agents of the present invention. Additional heat generating agents are such that; they also generate a heat by mixing with water, however, do not warm the composition to a temperature higher than the melting point of the phase changing agents. Such additional heat generating agents useful herein include, for example, organic heat generating agents such as polyhydric alcohols.

The polyhydric alcohol useful herein includes, for example, polyethylene glycol, polypropylene glycol, 1,2-propane diol or propylene glycol, 1,3-propane diol, hexylene glycol, glycerin, diethylene glycol, dipropylene glycol, 1,2-butylene glycol, 1,4-butylene glycol, and mixtures thereof. These polyhydric alcohols can also be used as the "INERT CARRIER" described above.

Such additional heat generating agents can be included in the compositions at a level by weight of, preferably from about 2% to about 85%, more preferably from about 5% to about 85%, still more preferably from about 10% to about 85%.

### HAIR CONDITIONING COMPOSITION

The anhydrous cosmetic compositions of the present invention are preferably anhydrous hair care compositions, more preferably anhydrous hair conditioning compositions. The anhydrous hair conditioning compositions preferably comprise hair conditioning agents in addition to the above described heat generating agent, the phase changing agent, and the inert carrier. The hair conditioning agents useful herein include, for example, high melting point fatty compounds, amidoamines, acids, cationic conditioning agents such as cationic surfactants and cationic polymers, low melting point oils, silicone compounds, polypropylene glycol, polyethylene glycol, and mixtures thereof. Among these hair conditioning agents, preferred are high melting point fatty compounds, amidoamines, acids, and mixtures thereof.

### HIGH MELTING POINT FATTY COMPOUND

The hair conditioning composition of the present invention preferably comprises a high melting point fatty compound. The high melting point fatty compound can be used as the "PHASE CHANGING AGENT" described above.

The high melting point fatty compound useful herein have a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The high melting point fatty compound can be included in the composition at a level by weight of, preferably from about 0.1% to about 30%, more preferably from about 0.2% to about 25%, still more preferably from about 0.5% to about 15%.

The fatty alcohols useful herein are those having from about 14 to about 30 carbon atoms, preferably from about 16 to about 22 carbon atoms. These fatty alcohols are saturated and can be straight or branched chain alcohols. Nonlimiting examples of fatty alcohols include, cetyl alcohol, stearyl alcohol, behenyl alcohol, and mixtures thereof.

The fatty acids useful herein are those having from about 10 to about 30 carbon atoms, preferably from about 12 to about 22 carbon atoms, and more preferably from about 16 to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, *i.e.* a mixture of fatty alcohols containing predominantly cetyl and stearyl alcohol, wherein the numeric designation indicates the number of ethylene glycol moieties present; C₁-C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

High melting point fatty compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago lllinois, USA), HYSTRENE available from Witco Corp. (Dublin Ohio, USA), and DERMA available from Vevy (Genova, Italy).

### AMIDOAMINE

The hair conditioning composition of the present invention preferably comprises an amidoamine of the following general formula:

R¹ CONH (CH₂)ₘ N (R²)₂

wherein R¹ is a residue of C₁₁ to C₂₄ fatty acids, R² is a C₁ to C₄ alkyl, and m is an integer from 1 to 4.

The amidoamine can be included in the composition at a level by weight of, preferably from about 0.05% to about 10%, more preferably from about 0.05% to about 8%, still more preferably from about 0.1 % to about 5%.

Preferred amidoamines useful in the present invention includes stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixtures thereof; more preferably stearamidopropyldimethylamine, stearamidoethyldiethylamine, and mixtures thereof.

Commercially available amidoamines useful herein include: stearamidopropyldimethylamine having tradename SAPDMA available from Inolex, and tradename Amidoamine MPS available from Nikko.

### ACIDS

The hair conditioning composition of the present invention preferably comprises an acid selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof; preferably ℓ-glutamic acid, lactic acid, hydrochloric acid, and mixtures thereof. The acid described herein can also be used as the "REACTION CONTROL AGENT" described above. The acid can be contained at a level such that the mole ratio of amidoamine to acid is, preferably from about 1:0.3 to about 1:1, more preferably from about 1:0.5 to about 1:0.9.

Commercially available acids useful herein include: ℓ-Glutamic acid: ℓ-Glutamic acid (cosmetic grade) available from Ajinomoto.

### CATIONIC CONDITIONING AGENT

The hair conditioning composition of the present invention may contain a cationic conditioning agent.

The cationic conditioning agent can be included in the composition at a level by weight of, preferably from about 0.1% to about 10%, more preferably from about 0.25% to about 8%, still more preferably from about 0.5% to about 3%.

The cationic conditioning agent is selected from the group consisting of cationic surfactants, cationic polymers, and mixtures thereof.

### Cationic surfactant

The cationic surfactant useful herein is any known to the artisan and described below.

Among the cationic surfactants useful herein are those corresponding to the general formula (I): 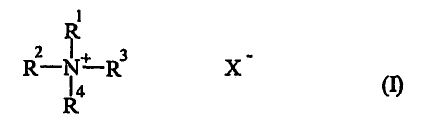 wherein at least one of R¹, R², R³, and R⁴ is selected from an aliphatic group of from 8 to 30 carbon atoms or an aromatic, alkoxy, polyoxyalkylene, alkylamido, hydroxyalkyl, aryl or alkylaryl group having up to about 22 carbon atoms, the remainder of R¹, R², R³, and R⁴ are independently selected from an aliphatic group of from 1 to about 22 carbon atoms or an aromatic, alkoxy, polyoxyalkylene, alkylamido, hydroxyalkyl, aryl or alkylaryl group having up to about 22 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The aliphatic groups can contain, in addition to carbon and hydrogen atoms, ether linkages, and other groups such as amino groups. The longer chain aliphatic groups, e.g., those of about 12 carbons, or higher, can be saturated or unsaturated. Preferred is when R¹, R², R³, and R⁴ are independently selected from C₁ to about C₂₂ alkyl. Nonlimiting examples of cationic surfactants useful in the present invention include the materials having the following CTFA designations: quaternium-8, quaternium-14, quaternium-18, quaternium-18 methosulfate, quatemium-24, and mixtures thereof.

Among the cationic surfactants of general formula (I), preferred are those containing in the molecule at least one alkyl chain having at least 16 carbons. Nonlimiting examples of such preferred cationic surfactants include: behenyl trimethyl ammonium chloride available, for example, with tradename INCROQUAT TMC-80 from Croda and ECONOL TM22 from Sanyo Kasei; cetyl trimethyl ammonium chloride available, for example, with tradename CA-2350 from Nikko Chemicals, hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methy) pyridinium chloride.

Also preferred are hydrophilically substituted cationic surfactants in which at least one of the substituents contain one or more aromatic, ether, ester, amido, or amino moieties present as substituents or as linkages in the radical chain, wherein at least one of the R¹ - R⁴ radicals contain one or more hydrophilic moieties selected from alkoxy (preferably C₁ - C₃ alkoxy), polyoxyalkylene (preferably C₁ - C₃ polyoxyalkylene), alkylamido, hydroxyalkyl, alkylester, and combinations thereof. Preferably, the hydrophilically substituted cationic conditioning surfactant contains from 2 to about 10 nonionic hydrophile moieties located within the above stated ranges. Preferred hydrophilically substituted cationic surfactants include those of the formula (II) through (VIII) below: 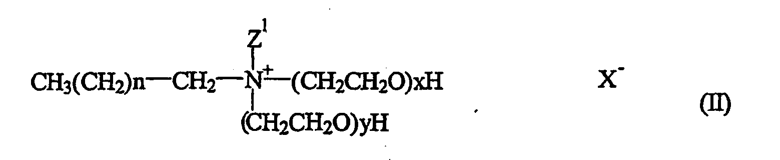 wherein n is from 8 to about 28, x+y is from 2 to about 40, Z¹ is a short chain alkyl, preferably a C₁ - C₃ alkyl, more preferably methyl, or (CH₂CH₂O)_{Z}H
wherein x+y+z is up to 60, and X is a salt forming anion as defined above; 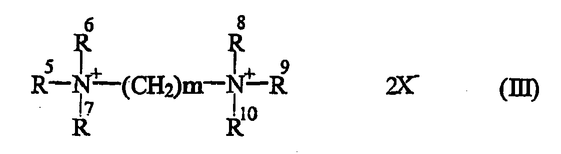 wherein m is 1 to 5, one or more of R⁵, R⁶, and R⁷ are independently an C₁ - C₃₀ alkyl, the remainder are CH₂CH₂OH, one or two of R⁸, R⁹, and R¹⁰ are independently an C₁ - C₃₀ alkyl, and remainder are CH₂CH₂OH, and X is a salt forming anion as mentioned above; 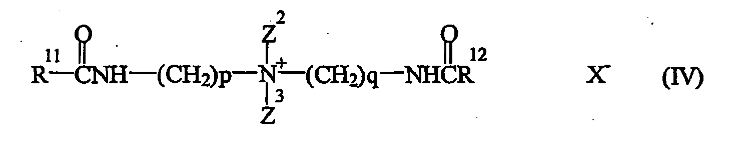 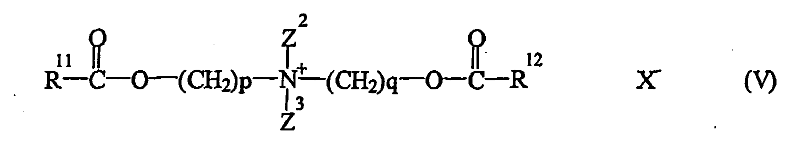 wherein, independently for formulae (IV) and (V), Z² is an alkyl, preferably a C₁ - C₃ alkyl, more preferably methyl, and Z³ is a short chain hydroxyalkyl, preferably hydroxymethyl or hydroxyethyl, p and q independently are integers from 2 to 4, inclusive, preferably from 2 to 3, inclusive, more preferably 2, R¹¹ and R¹² , independently, are substituted or unsubstituted hydrocarbyls, preferably C₁₂ - C₂₀ alkyl or alkenyl, and X is a salt forming anion as defined above; 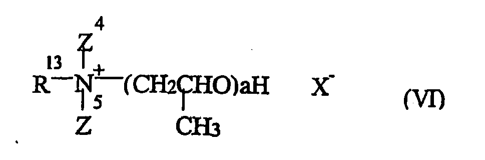 wherein R¹³ is a hydrocarbyl, preferably a C₁ - C₃ alkyl, more preferably methyl, Z⁴ and Z⁵ are, independently, short chain hydrocarbyls, preferably C₂ - C₄ alkyl or alkenyl, more preferably ethyl, a is from 2 to about 40, preferably from about 7 to about 30, and X is a salt forming anion as defined above; 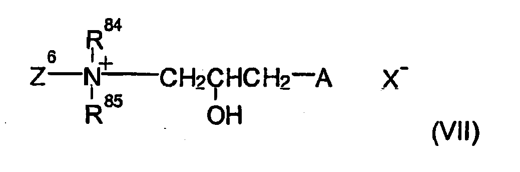 wherein R⁸⁴ and R⁸⁵, independently, are C₁ - C₃ alkyl, preferably methyl, Z⁶ is a C₁₂ - C₂₂ hydrocarbyl, alkyl carboxy or alkylamido, and A is a protein, preferably a collagen, keratin, milk protein, silk, soy protein, wheat protein, or hydrolyzed forms thereof; and X is a salt forming anion as defined above; 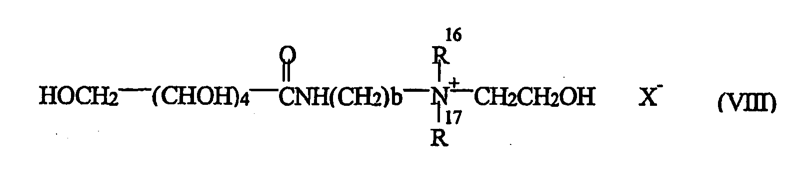 wherein b is 2 or 3, R¹⁶ and R¹⁷, independently are C₁ - C₃ hydrocarbyls preferably methyl, and X is a salt forming anion as defined above. Nonlimiting examples of hydrophilically substituted cationic surfactants useful in the present invention include the materials having the following CTFA designations:
quaternium-16, quaternium-26, quaternium-27, quaternium-30, quaternium-33, quaternium-43, quaternium-52, quaternium-53, quaternium-56, quaternium-60, quaternium-61, quaternium-62, quaternium-70, quaternium-71, quaternium-72, quaternium-75, quaternium-76 hydrolyzed collagen, quaternium-77, quaternium-78, quaternium-79 hydrolyzed collagen, quaternium-79 hydrolyzed keratin, quaternium-79 hydrolyzed milk protein, quaternium-79 hydrolyzed silk, quaternium-79 hydrolyzed soy protein, and quaternium-79 hydrolyzed wheat protein, quaternium-80, quaternium-81, quaternium-82, quaternium-83, quaternium-84, and mixtures thereof.

Highly preferred hydrophilically substituted cationic surfactants include dialkylamido ethyl hydroxyethylmonium salt, dialkylamidoethyl dimonium salt, dialkyloyl ethyl hydroxyethylmonium salt, dialkyloyl ethyldimonium salt, and mixtures thereof; for example, commerically available under the following tradenames; VARISOFT 110, VARIQUAT K1215 and 638 from Witco Chemical, MACKPRO KLP, MACKPRO WLW, MACKPRO MLP, MACKPRO NSP, MACKPRO NLW, MACKPRO WWP, MACKPRO NLP, MACKPRO SLP from Mclntyre, ETHOQUAD 18/25, ETHOQUAD O/12PG, ETHOQUAD C/25, ETHOQUAD S/25, and ETHODUOQUAD from Akzo, DEHYQUAT SP from Henkel, and ATLAS G265 from ICI Americas.

### Cationic Polymer

The cationic polymer useful herein is described below. As used herein, the term "polymer" shall include materials whether made by polymerization of one type of monomer or made by two (i.e., copolymers) or more types of monomers.

Preferably, the cationic polymer is a water-soluble cationic polymer. By "water soluble" cationic polymer, what is meant is a polymer which is sufficiently soluble in water to form a substantially clear solution to the naked eye at a concentration of 0.1% in water (distilled or equivalent) at 25°C. The preferred polymer will be sufficiently soluble to form a substantially clear solution at 0.5% concentration, more preferably at 1.0% concentration.

The cationic polymers hereof will generally have a weight average molecular weight which is at least about 5,000, typically at least about 10,000, and is less than about 10 million. Preferably, the molecular weight is from about 100,000 to about 2 million. The cationic polymers will generally have cationic nitrogen-containing moieties such as quaternary ammonium or cationic amino moieties, and mixtures thereof.

The cationic charge density is preferably at least about 0.1 meq/gram, more preferably at least about 1.5 meq/gram, even more preferably at least about 1.1 meq/gram, still more preferably at least about 1.2 meq/gram. Cationic charge density of the cationic polymer can be determined according to the Kjeldahl Method. Those skilled in the art will recognize that the charge density of amino-containing polymers may vary depending upon pH and the isoelectric point of the amino groups. The charge density should be within the above limits at the pH of intended use.

Any anionic counterions can be utilized for the cationic polymers so long as the water solubility criteria is met. Suitable counterions include halides (e.g., Cl, Br, I, or F, preferably Cl, Br, or I), sulfate, and methylsulfate. Others can also be used, as this list is not exclusive.

The cationic nitrogen-containing moiety will be present generally as a substituent, on a fraction of the total monomer units of the cationic hair conditioning polymers. Thus, the cationic polymer can comprise copolymers, terpolymers, etc. of quaternary ammonium or cationic amine-substituted monomer units and other non-cationic units referred to herein as spacer monomer units. Such polymers are known in the art, and a variety can be found in the CTFA Cosmetic Ingredient Dictionary, 3rd edition, edited by Estrin, Crosley, and Haynes, (The Cosmetic, Toiletry, and Fragrance Association, Inc., Washington, D.C., 1982).

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. The alkyl and dialkyl substituted monomers preferably have C₁ - C₇ alkyl groups, more preferably C₁ - C₃ alkyl groups. Other suitable spacer monomers include vinyl esters, vinyl alcohol (made by hydrolysis of polyvinyl acetate), maleic anhydride, propylene glycol, and ethylene glycol.

The cationic amines can be primary, secondary, or tertiary amines, depending upon the particular species and the pH of the composition. In general, secondary and tertiary amines, especially tertiary amines, are preferred.

Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R'X wherein R' is a short chain alkyl, preferably a C₁- C₇ alkyl, more preferably a C₁ - C₃ alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts. The alkyl portions of these monomers are preferably lower alkyls such as the C₁ - C₃ alkyls, more preferably C₁ and C₂ alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C₁ - C₇ hydrocarbyls, more preferably C₁- C₃, alkyls.

The cationic polymers hereof can comprise mixtures of monomer units derived from amine- and/or quaternary ammonium-substituted monomer and/or compatible spacer monomers.

Suitable cationic hair conditioning polymers include, for example: copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT tradename (e.g., LUVIQUAT FC 370); copolymers of 1-vinyl-2-pyrrolidone and dimethylaminoethyl methacrylate (referred to in the industry by CTFA as Polyquaternium-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; and mineral acid salts of amino-alkyl esters of homo- and co-polymers of unsaturated carboxylic acids having from 3 to 5 carbon atoms, as described in U.S. Patent 4,009,256, incorporated herein by reference.

Other cationic polymers that can be used include polysaccharide polymers, such as cationic cellulose derivatives and cationic starch derivatives.

Cationic polysaccharide polymer materials suitable for use herein include those of the formula: 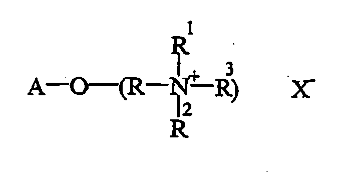 wherein: A is an anhydroglucose residual group, such as a starch or cellulose anhydroglucose residual, R is an alkylene oxyalkylene, polyoxyalkylene, or hydroxyalkylene group, or combination thereof, R¹, R², and R³ independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R¹, R² and R³) preferably being about 20 or less, and X is an anionic counterion, as previously described.

Cationic cellulose is available from Amerchol Corp. (Edison, NJ, USA) in their Polymer JR® and LR® series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200®.

Other cationic polymers that can be used include cationic guar gum derivatives, such as guar hydroxypropyltrimonium chloride (commercially available from Celanese Corp. in their Jaguar R series). Other materials include quaternary nitrogen-containing cellulose ethers (e.g., as described in U.S. Patent 3,962,418, incorporated herein by reference), and copolymers of etherified cellulose and starch (e.g., as described in U.S. Patent 3,958,581).

### LOW MELTING POINT OIL

The hair conditioning composition of the present invention may contain a low melting point oil, which has a melting point of less than 25°C. The low melting point oil can be included in the composition at a level by weight of, preferably from about 0.1% to about 10%, more preferably from about 0.25% to about 6%. The low melting point oil can be used as the "INERT CARRIER" described above.

The low melting point oil useful herein is selected from the group consisting of hydrocarbon having from 10 to about 40 carbon atoms, unsaturated fatty alcohols having from about 10 to about 30 carbon atoms, unsaturated fatty acids having from about 10 to about 30 carbon atoms, fatty acid derivatives, fatty alcohol derivatives, ester oils, poly α-olefin oils, and mixtures thereof.

Fatty alcohols useful herein include those having from about 10 to about 30 carbon atoms, preferably from about 12 to about 22 carbon atoms, and more preferably from about 16 to about 22 carbon atoms. These fatty alcohols are unsaturated and can be straight or branched chain alcohols. Suitable fatty alcohols include, for example, oleyl alcohol, isostearyl alcohol, tridecylalcohol, decyl tetradecyl alcohol, and octyl dodecyl alcohol. These alcohols are available, for example, from Shinnihon Rika.

Low melting point oils useful herein include pentaerythritol ester oils, trimethylol ester oils, poly α-olefin oils, citrate ester oils, glyceryl ester oils, and mixtures thereof, and the ester oil useful herein is water-insoluble. As used herein, the term "water-insoluble" means the compound is substantially not soluble in water at 25°C; when the compound is mixed with water at a concentration by weight of above 1.0%, preferably at above 0.5%, the compound is temporarily dispersed to form an unstable colloid in water, then is quickly separated from water into two phases.

Pentaerythritol ester oils useful herein are those having the following formula: 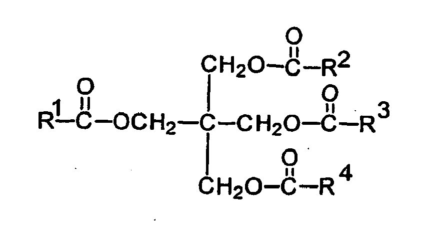 wherein R¹, R², R³, and R⁴, independently, are branched, straight, saturated, or unsaturated alkyl, aryl, and alkylaryl groups having from 1 to about 30 carbons. Preferably, R¹, R², R³, and R⁴, independently, are branched, straight, saturated, or unsaturated alkyl groups having from about 8 to about 22 carbons. More preferably, R¹, R², R³ and R⁴ are defined so that the molecular weight of the compound is from about 800 to about 1200.

Trimethylol ester oils useful herein are those having the following formula: 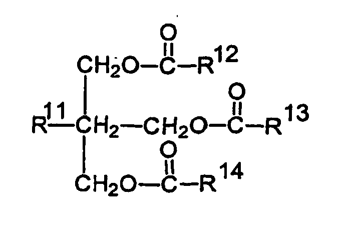 wherein R¹¹ is an alkyl group having from 1 to about 30 carbons, and R¹², R¹³, and R¹⁴, independently, are branched, straight, saturated, or unsaturated alkyl, aryl, and alkylaryl groups having from 1 to about 30 carbons. Preferably, R¹¹ is ethyl and R¹², R¹³, and R¹⁴, independently, are branched, straight, saturated, or unsaturated alkyl groups having from 8 to about 22 carbons. More preferably, R¹¹, R¹², R¹³ and R¹⁴ are defined so that the molecular weight of the compound is from about 800 to about 1200.

Particularly useful pentaerythritol ester oils and trimethylol ester oils herein include pentaerythritol tetraisostearate, pentaerythritol tetraoleate, trimethylolpropane triisostearate, trimethylolpropane trioleate, and mixtures thereof. Such compounds are available from Kokyo Alcohol with tradenames KAKPTI, KAKTTI, and Shin-nihon Rika with tradenames PTO, ENUJERUBU TP3SO.

Poly α-olefin oils useful herein are those derived from 1-alkene monomers having from about 6 to about 16 carbons, preferably from about 6 to about 12 carbons atoms. Nonlimiting examples of 1-alkene monomers useful for preparing the poly α-olefin oils include 1-hexene, 1-octene, 1-decene, 1-dodecene, 1-tetradecene, 1-hexadecene, branched isomers such as 4-methyl-1-pentene, and mixtures thereof. Preferred 1-alkene monomers useful for preparing the poly α-olefin oils are 1-octene, 1-decene, 1-dodecene, 1-tetradecene, 1-hexadecene, and mixtures thereof. Poly α-olefin oils useful herein further have a viscosity of from about 1 to about 35,000 cst, a molecular weight of from about 200 to about 60,000, and a polydispersity of no more than about 3.

Poly α-olefin oils having a molecular weight of at least about 800 are useful herein. Such high molecular weight poly α-olefin oils are believed to provide long lasting moisturized feel to the hair. Poly α-olefin oils having a molecular weight of less than about 800 are useful herein. Such low molecular weight poly α-olefin oils are believed to provide a smooth, light, clean feel to the hair.

Particularly useful poly α-olefin oils herein include polydecenes with tradenames PURESYN 6 having a number average molecular weight of about 500 and PURESYN 100 having a number average molecular weight of about 3000 and PURESYN 300 having a number average molecular weight of about 6000 available from Mobil Chemical Co.

Citrate ester oils useful herein are those having a molecular weight of at least about 500 having the following formula: 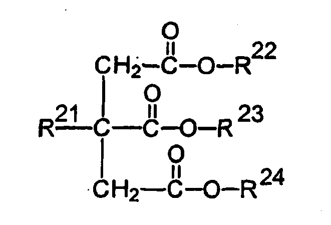 wherein R²¹ is OH or CH₃COO, and R²², R²³, and R²⁴, independently, are branched, straight, saturated, or unsaturated alkyl, aryl, and alkylaryl groups having from 1 to about 30 carbons. Preferably, R²¹ is OH, and R²², R²³, and R²⁴, independently, are branched, straight, saturated, or unsaturated alkyl, aryl, and alkylaryl groups having from 8 to about 22 carbons. More preferably, R²¹, R²², R²³ and R²⁴ are defined so that the molecular weight of the compound is at least about 800.

Particularly useful citrate ester oils herein include triisocetyl citrate with tradename CITMOL 316 available from Bernel, triisostearyl citrate with tradename PELEMOL TISC available from Phoenix, and trioctyldodecyl citrate with tradename CITMOL 320 available from Bernel. Glyceryl ester oils useful herein are those having a molecular weight of at least about 500 and having the following formula: 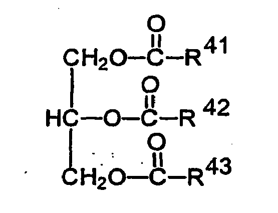 wherein R⁴¹, R⁴², and R⁴³, independently, are branched, straight, saturated, or unsaturated alkyl, aryl, and alkylaryl groups having from 1 to about 30 carbons. Preferably, R⁴¹, R⁴², and R⁴³, independently, are branched, straight, saturated, or unsaturated alkyl, aryl, and alkylaryl groups having from 8 to about 22 carbons. More preferably, R⁴¹, R⁴², and R⁴³ are defined so that the molecular weight of the compound is at least about 800.

Particularly useful glyceryl ester oils herein include triisostearin with tradename SUN ESPOL G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks.

### SILICONE COMPOUNDS

The hair conditioning composition of the present invention may contain silicone compound. The silicone compound can be included in the composition at a level by weight of, preferably from about 0.1% to about 10%, more preferably from about 0.25% to about 8%, still more preferably from about 0.5% to about 3%.

The silicone compounds hereof can include volatile soluble or insoluble, or nonvolatile soluble or insoluble silicone conditioning agents. By soluble what is meant is that the silicone compound is miscible with the carrier of the composition so as to form part of the same phase. By insoluble what is meant is that the silicone forms a separate, discontinuous phase from the carrier, such as in the form of an emulsion or a suspension of droplets of the silicone. The silicone compounds herein may be made by conventional polymerization, or emulsion polymerization.

The silicone compounds for use herein will preferably have a viscosity of from about 1,000 to about 2,000,000 centistokes at 25°C, more preferably from about 10,000 to about 1,800,000, and even more preferably from about 25,000 to about 1,500,000. The viscosity can be measured by means of a glass capillary viscometer as set forth in Dow Corning Corporate Test Method CTM0004, July 20, 1970, which is incorporated by reference herein in its entirety. Silicone compound of high molecular weight may be made by emulsion polymerization.

Silicone compounds useful herein include polyalkyl polyaryl siloxanes, polyalkyleneoxide-modified siloxanes, silicone resins, amino-substituted siloxanes, and mixtures thereof. The silicone compound is preferably selected from the group consisting of polyalkyl polyaryl siloxanes, polyalkyleneoxide-modified siloxanes, silicone resins, and mixtures thereof, and more preferably from one or more polyalkyl polyaryl siloxanes.

Polyalkyl polyaryl siloxanes useful here in include those with the following structure (I) 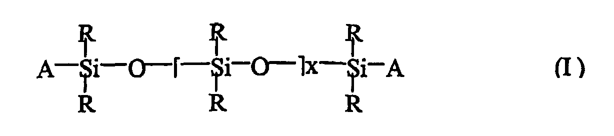 wherein R is alkyl or aryl, and x is an integer from about 7 to about 8,000. "A" represents groups which block the ends of the silicone chains. The alkyl or aryl groups substituted on the siloxane chain (R) or at the ends of the siloxane chains (A) can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable A groups include hydroxy, methyl, methoxy, ethoxy, propoxy, and aryloxy. The two R groups on the silicon atom may represent the same group or different groups. Preferably, the two R groups represent the same group. Suitable R groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from the General Electric Company in their ViscasilR and SF 96 series, and from Dow Corning in their Dow Corning 200 series. Polymethylphenylsiloxanes, for example, from the General Electric Company as SF 1075 methyl phenyl fluid or from Dow Corning as 556 Cosmetic Grade Fluid, are useful herein.

Also preferred, for enhancing the shine characteristics of hair, are highly arylated silicone compounds, such as highly phenylated polyethyl silicone having refractive index of about 1.46 or higher, especially about 1.52 or higher. When these high refractive index silicone compounds are used, they should be mixed with a spreading agent, such as a surfactant or a silicone resin, as described below to decrease the surface tension and enhance the film forming ability of the material.

Another polyalkyl polyaryl siloxane that can be especially useful is a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. Silicone gums are described by Petrarch, and others including U.S. Patent No. 4,152,416, to Spitzer et al., issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copoiymer and mixtures thereof.

Polyalkyleneoxide-modified siloxanes useful herein include, for example, polypropylene oxide modified and polyethylene oxide modified polydimethylsiloxane. The ethylene oxide and polypropylene oxide level should be sufficiently low so as not to interfere with the dispersibility characteristics of the silicone. These material are also known as dimethicone copolyols.

Silicone resins, which are highly crosslinked polymeric siloxane systems, are useful herein. The crosslinking is introduced through the incorporation of trifunctional and tetra-functional silanes with mono-functional or di-functional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units, and hence, a sufficient level of crosslinking, such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. Preferably, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinylchlorosilanes, and tetrachlorosilane, with the methyl substituted silanes being most commonly utilized. Preferred resins are offered by General Electric as GE SS4230 and SS4267. Commercially available silicone resins will generally be supplied in a dissolved form in a low viscosity volatile or nonvolatile silicone fluid. The silicone resins for use herein should be supplied and incorporated into the present compositions in such dissolved form, as will be readily apparent to those skilled in the art. Without being bound by theory, it is believed that the silicone resins can enhance deposition of other silicone compounds on the hair and can enhance the glossiness of hair with high refractive index volumes.

Other useful silicone resins are silicone resin powders such as the material given the CTFA designation polymethylsilsequioxane, which is commercially available as Tospearl^{™} from Toshiba Silicones.

Silicone resins can conveniently be identified according to a shorthand nomenclature system well known to those skilled in the art as the "MDTQ" nomenclature. Under this system, the silicone is described according to the presence of various siloxane monomer units which make up the silicone. Briefly, the symbol M denotes the mono-functional unit (CH₃)₃SiO)_{.5}; D denotes the difunctional unit (CH₃)₂SiO; T denotes the trifunctional unit (CH₃)SiO_{1,5}; and Q denotes the quadri- or tetra-functional unit SiO₂. Primes of the unit symbols, e.g., M', D', T', and Q' denote substituents other than methyl, and must be specifically defined for each occurrence. Typical alternate substituents include groups such as vinyl, phenyl, amino, hydroxyl, etc. The molar ratios of the various units, either in terms of subscripts to the symbols indicating the total number of each type of unit in the silicone, or an average thereof, or as specifically indicated ratios in combination with molecular weight, complete the description of the silicone material under the MDTQ system. Higher relative molar amounts of T, Q, T and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5:1.0 to about 1.5:1.0 and the average molecular weight of the resin is from about 9000 to about 10,000.

Amino-substituted siloxanes useful herein include those represented by the following structure (II) 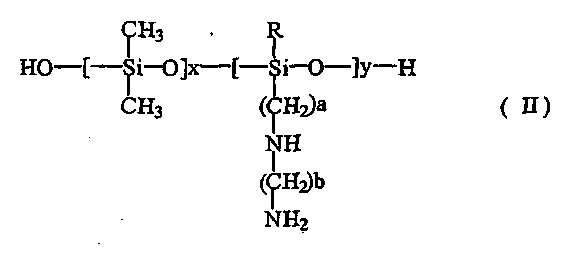 wherein R is CH₃ or OH, x and y are integers which depend on the molecular weight, the average molecular weight being approximately between 5,000 and 10,000. This polymer is also known as "amodimethicone".

Suitable amino-substituted siloxane fluids include those represented by the formula (III)

(R₁)ₐG₃₋ₐ-Si-(-OSiG₂)ₙ-(-OSiG_{b}(R₁)_{2-b})ₘ-O-SiG₃₋ₐ(R₁)ₐ (III)

in which G is chosen from the group consisting of hydrogen, phenyl, OH, C₁-C₈ alkyl and preferably methyl; a denotes 0 or an integer from 1 to 3, and preferably equals 0; b denotes 0 or 1 and preferably equals 1; the sum n+m is a number from 1 to 2,000 and preferably from 50 to 150, n being able to denote a number from 0 to 1,999 and preferably from 49 to 149 and m being able to denote an integer from 1 to 2,000 and preferably from 1 to 10; R₁ is a monovalent radical of formula CqH_{2q}L in which q is an integer from 2 to 8 and L is chosen from the groups

-N(R₂)CH₂-CH₂-N(R₂)₂

-N(R₂)₂

-N(R₂)₃A⁻

-N(R₂)CH₂-CH₂-NR₂H₂A⁻

in which R₂ is chosen from the group consisting of hydrogen, phenyl, benzyl, a saturated hydrocarbon radical, preferably an alkyl radical containing from 1 to 20 carbon atoms, and A⁻ denotes a halide ion.

An especially preferred amino-substituted siloxane corresponding to formula (III) is the polymer known as "trimethylsilylamodimethicone", of formula (IV):

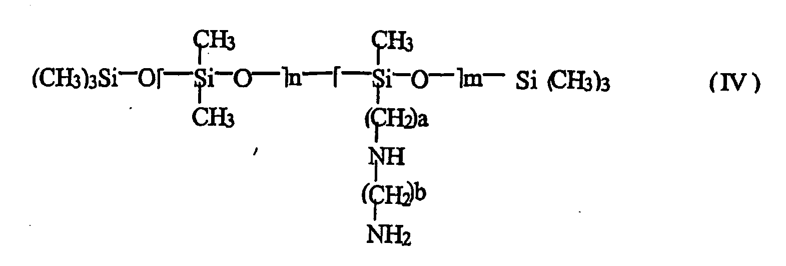

In this formula n and m are selected depending on the molecular weight of the compound desired.

Other amino-substituted siloxane which can be used are represented by the formula (V): 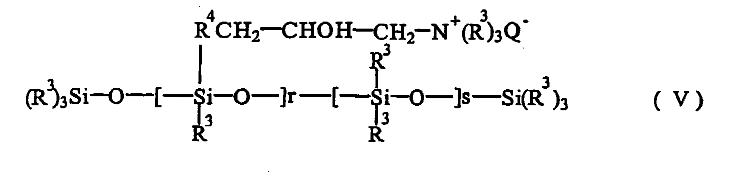 where R³ denotes a monovalent hydrocarbon radical having from 1 to 18 carbon atoms, preferably an alkyl or alkenyl radical such as methyl; R₄ denotes a hydrocarbon radical, preferably a C₁ - C₁₈ alkylene radical or a C₁ - C₁₈, and more preferably C₁ - C₈, alkyleneoxy radical; Q⁻ is a halide ion, preferably chloride; r denotes an average statistical value from 2 to 20, preferably from 2 to 8; s denotes an average statistical value from 20 to 200, and preferably from 20 to 50. A preferred polymer of this class is available from Union Carbide under the name "UCAR SILICONE ALE 56."

### POLYPROPYLENE GLYCOL

The hair conditioning composition of the present invention may contain a polypropylene glycol as a conditioning agent. The polypropylene glycol can be included in the composition at a level by weight of, preferably from about 0.1 % to about 10%, more preferably from about 0.25% to about 6%. Some polypropylene glycol described herein can also be used as the "VISCOSITY MODIFYING AGENT" described above.

The polypropylene glycol useful herein may has a weight average molecular weight of preferably from about 200 g/mol to about 100,000 g/mol, more preferably from about 1,000 g/mol to about 60,000 g/mol. Without intending to be limited by theory, it is believed that the polypropylene glycol herein deposits onto, or is absorbed into hair to act as a moisturizer buffer, and/or provides one or more other desirable hair conditioning benefits. As used herein, the term "polypropylene glycol" includes single-polypropylene glycol-chain segment polymers, and multi-polypropylene glycol-chain segment polymers. The general structure of branched polymers such as the multi-polypropylene glycol-chain segment polymers herein are described, for example, in "Principles of Polymerization," pp. 17-19, G. Odlan, (John Wiley & Sons, Inc., 3^{rd} ed., 1991).

The polypropylene glycol herein are typically polydisperse polymers. The polypropylene glycols useful herein have a polydispersity of from about 1 to about 2.5, preferably from about 1 to about 2, and more preferably from about 1 to about 1.5. As used herein, the term "polydispersity" indicates the degree of the molecular weight distribution of the polymer sample. Specifically, the polydispersity is a ratio, greater than 1, equal to the weight average molecular weight divided by the number average molecular weight. For a further discussion about polydispersity, see "Principles of Polymerization," pp. 20-24, G. Odian, (John Wiley & Sons, Inc., 3^{rd} ed., 1991).

The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form (e.g., leave-on, or rinse-off form) of the hair care composition. The solubility in water of the polypropylene glycol herein may be chosen by the artisan according to a variety of factors. Accordingly, for a leave-on hair care composition, it is preferred that the polypropylene glycol herein be a water-soluble polypropylene glycol. Solubility information is readily available from polypropylene glycol suppliers, such as Sanyo Kasei (Osaka, Japan). However, the present invention may also take the form of a rinse-off hair care composition. Without intending to be limited by theory, it is believed that in such a composition, a water-soluble polypropylene glycol may be too easily washed away before it effectively deposits on hair and provides the desired benefit(s). For such a composition, a less soluble, or even a water-insoluble polypropylene glycol is therefore preferred. Accordingly, for a rinse-off hair care composition, it is preferred that the polypropylene glycol herein has a solubility in water at 25 °C of less than about 1 g/100 g water, more preferably a solubility in water of less than about 0.5 g/100 g water, and even more preferably a solubility in water of less than about 0.1 g/100 g water.

Preferably the polypropylene glycol is selected from the group consisting of a single-polypropylene glycol-chain segment polymer, a multi-polypropylene glycol-chain segment polymer, and mixtures thereof, more preferably selected from the group consisting of a single-polypropylene glycol-chain segment polymer of Formula I, below, a multi-polypropylene glycol-chain segment polymer of Formula II, below, and mixtures thereof.

### Single-Polypropylene Glycol-Chain Segment Polymer

Accordingly, a highly preferred single-polypropylene glycol-chain segment polymer has the formula:

HO-(C₃H₆O)ₐH (III),

wherein a is a value from about 4 to about 400, preferably from about 20 to about 100, and more preferably from about 20 to about 40.

The single-polypropylene glycol-chain segment polymer useful herein is typically inexpensive, and is readily available from, for example, Sanyo Kasei (Osaka, Japan), Dow Chemicals (Midland, Michigan, USA), Calgon Chemical, Inc. (Skokie, Illinois, USA), Arco Chemical Co. (Newton Square Pennsylvania, USA), Witco Chemicals Corp. (Greenwich, Connecticut, USA), and PPG Specialty Chemicals (Gurnee, Illinois, USA).

### Multi-Polypropylene Glycol-Chain Segment Polymer

A highly preferred multi-polypropylene glycol-chain segment polymer has the formula: 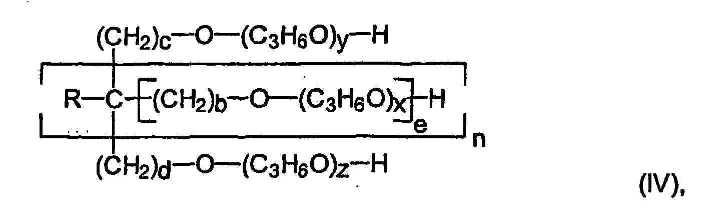 wherein n is a value from about 0 to about 10, preferably from about 0 to about 7, and more preferably from about 1 to about 4. In Formula IV, each R" is independently selected from the group consisting of H, and C₁-C₃₀ alkyl, and preferably each R" is independently selected from the group consisting of H, and C₁-C₄ alkyl. In Formula IV, each b is independently a value from about 0 to about 2, preferably from about 0 to about 1, and more preferably b = 0. Similarly, c and d are independently a value from about 0 to about 2, preferably from about 0 to about 1. However, the total of b + c + d is at least about 2, preferably the total of b + c + d is from about 2 to about 3. Each e is independently a value of 0 or 1, if n is from about 1 to about 4, then e is preferably equal to 1. Also in Formula IV, x, y, and z is independently a value of from about 1 to about 120, preferably from about 7 to about 100, and more preferably from about 7 to about 100, where x + y + z is greater than about 20.

Examples of the multi-polypropylene glycol-chain segment polymer of Formula IV which is especially useful herein includes polyoxypropylene glyceryl ether (n = 1, R' = H, b = 0, c and d = 1, e = 1, and x, y, and z independently indicate the degree of polymerization of their respective polypropylene glycol-chain segments; available as New Pol GP-4000, from Sanyo Kasei, Osaka, Japan), polypropylene trimethylol propane (n = 1, R' = C₂H₅, b = 1, c and d = 1, e = 1, and x, y, and z independently indicate the degree of polymerization of their respective polypropylene glycol-chain segments), polyoxypropylene sorbitol (n = 4, each R' = H, b = 0, c and d = 1, each e = 1, and y, z, and each x independently indicate the degree of polymerization of their respective polypropylene glycol-chain segments; available as New Pol SP-4000, from Sanyo Kasei, Osaka, Japan), and PPG-10 butanediol (n = 0, c and d = 2, and y + z = 10; available as Probutyl DB-1 0, from Croda, Inc., of Parsippany, New Jersey, U.S.A.).

In a preferred embodiment, one or more of the propylene repeating groups in the polypropylene glycol is an isopropyl oxide repeating group. More preferably one or more of the propylene oxide repeating groups of the polypropylene glycol of Formula III and/or the polypropylene glycol of Formula IV is an isopropyl oxide repeating group. Even more preferably, substantially all of the propylene oxide repeating groups of the polypropylene glycol of Formula III and/or the polypropylene glycol of Formula IV are isopropyl oxide repeating groups. Accordingly, a highly preferred single-polypropylene glycol-chain segment polymer has the formula: 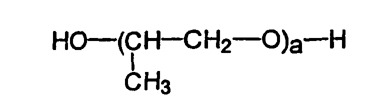 wherein a is defined as described above for Formula III. Similarly, a highly preferred multi-polypropylene glycol-chain segment polymer has the formula: 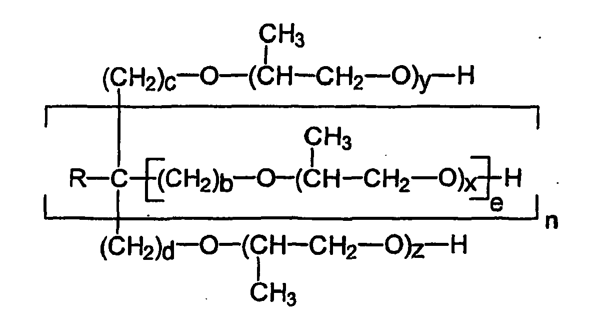 wherein n, R", b, c, d, e, x, y, and z are defined as above, for Formula IV. It is recognized that the isopropyl oxide repeating groups may also correspond either alone, or in combination with the above depicted, to:

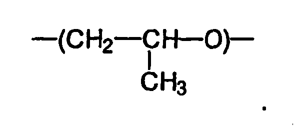

The polypropylene glycol useful herein is readily available from, for example, Sanyo Kasei (Osaka, Japan) as New pol PP-2000, New pol PP-4000, New pol GP-4000, and New pol SP-4000, from Dow Chemicals (Midland, Michigan, USA), from Calgon Chemical, Inc. (Skokie, Illinois, USA), from Arco Chemical Co. (Newton Square Pennsylvania, USA), from Witco Chemicals Corp. (Greenwich, Connecticut, USA), and from PPG Specialty Chemicals (Gurnee, Illinois, USA).

### HIGH MOLECULAR WEIGHT POLYETHYLENE GLYCOL

The hair conditioning composition of present invention may contain a high molecular weight polyethylene glycol as a conditioning agent. The high molecular weight polyethylene glycol can also be used as the "VISCOSITY MODIFYING AGENT" described above. The polyethylene glycols useful herein are those having the formula:

H(OCH₂CH₂)ₙ -OH

wherein n has an average value of from 2,000 to 14,000, preferably from about 5,000 to about 9,000, more preferably from about 6,000 to about 8,000.

The polyethylene glycol can be included in the composition at a level by weight of, preferably from about 0.1% to about 10%, more preferably from about 0.25% to about 6%.

The polyethylene glycol described above is also known as a polyethylene oxide, and polyoxyethylene. Polyethylene glycols useful herein that are especially preferred are PEG-2M wherein n has an average value of about 2,000 (PEG-2M is also known as Polyox WSR® N-10 from Union Carbide and as PEG-2,000); PEG-5M wherein n has an average value of about 5,000 (PEG-5M is also known as Polyox WSR® N-35 and as Polyox WSR® N-80, both from Union Carbide and as PEG-5,000 and Polyethylene Glycol 300,000); PEG-7M wherein n has an average value of about 7,000 (PEG-7M is also known as Polyox WSR® N-750 from Union Carbide); PEG-9M wherein n has an average value of about 9,000 (PEG-9M is also known as Polyox WSR® N-3333 from Union Carbide); and PEG-14M wherein n has an average value of about 14,000 (PEG-14M is also known as Polyox WSR® N-3000 from Union Carbide).

### PREFERRED HAIR CONDITIONING COMPOSITIONS

In one embodiment of the present invention, the anhydrous hair conditioning composition comprises by weight:
(a) from about 5% to about 60% of the heat generating agent which generates a heat by mixing with water, preferably an inorganic heat generating agent;
(b) from about 0.1 % to about 30% of the phase changing agent, preferably fatty compound, more preferably fatty alcohol selected from the group consisting of cetyl alcohol, stearyl alcohol, and mixtures thereof;
(c) from about 0.1 % to about 10% of the polyoxyalkylene derivative, preferably polyoxyethylene/polyoxypropylene block copolymer;
(d) from about 0.05% to about 10% of the amidoamine, preferably, the amidoamine selected from the group consisting of stearamidopropyl dimethylamine, stearamidoethyl diethylamine, and mixtures thereof;
(e) the acid at a level such that the mole ratio of the amidoamine to the acid is from about 1:0.3 to about 1:1, preferably, ℓ-Glutamic acid at a level such that the mole ratio of amidoamine to acid is from about 1:0.5 to about 1:0.9; and
(f) from about 10% to about 90% of the inert carrier, preferably, polyethylene glycol.

### ADDITIONAL COMPONENTS

The hair conditioning composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, a mixture of Polysorbate 60 and Cetearyl Alcohol with tradename Polawax NF available from Croda Chemicals, glycerylmonostearate available from Stepan Chemicals, hydroxyethyl cellulose available from Aqualon, 3-pyridinecarboxy acid amide (niacinamide), hydrolysed keratin, proteins, plant extracts, and nutrients; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; and sequestering agents, such as disodium ethylenediamine tetra-acetate; ultraviolet and infrared screening and absorbing agents such as octyl salicylate, antidandruff agents such as zinc pyridinethione, and salicylic acid; and optical brighteners, for example polystyrylstilbenes, triazinstilbenes, hydroxycoumarins, aminocoumarins, triazoles, pyrazolines, oxazoles, pyrenes, porphyrins, imidazoles, and mixtures thereof.

Non-heat generating particles can be formulated into the present compositions. The particle can be included in the compositions at a level by weight of, preferably from about 0.01% to about 10%, more preferably from about 0.1% to about 5%, still more preferably from about 0.1 % to about 2%. The particles useful herein has an average particle size of preferably from about 25µm to about 1500µm, more preferably from about 50µm to about 1000µm, still more preferably from about 50µm to about 500µm. Both organic and inorganic particles can be used herein. Preferred particles useful herein include organic particles such as cellulose particles, and inorganic particles such as mica, silica, mud, clay, and mixtures thereof. More preferred is silica. Some non-heat generating particles described herein can also be used as the "VISCOSITY MODIFYING AGENT" described above. Preferred particles useful herein can be those having a breakability such that the particles are breakable when the particles contained in the compositions are spread on the hands and/or on the hair. Commercially available particles useful herein include: silica having tradename Neosil series such as Neosil CBT 60 available from Crosfield.

### EXAMPLES

The following examples further describe and demonstrate embodiments within the scope of the present invention. Ingredients are identified by chemical or CTFA name, or otherwise defined below.

| Hair Conditioning Compositions | | | |
|---|---|---|---|
| Components | Ex.1 | Ex. 2 | Ex. 3 |
| Anhydrous magnesium sulfate (MgSO₄) | 35 | 25 | 45 |
| Polyethylene/polypropylene block copolymer *1 | 3.0 | 1.0 | 5.0 |
| Cetyl Alcohol *4 | 1.0 | 2.0 | 0.25 |
| Stearyl Alcohol *5 | 1.4 | 2.8 | 0.45 |
| Stearamidopropyl Dimethylamine *6 | 0.8 | 1.6 | 0.2 |
| ℓ-Glutamic acid *7 | 0.256 | 0.512 | 0.064 |
| Hydroxyethylcellulose *8 | - | 2.0 | 0.5 |
| Perfume | 0.3 | 0.3 | 0.3 |
| Benzyl alcohol | - | - | 0.4 |
| EDTA | - | - | 0.1 |
| Kathon CG *9 | - | - | 0.0005 |
| Sodium Chloride | - | - | 0.01 |
| 3-pyridinecarboxy acid amide | 0.05 | 0.05 | 0.05 |
| dl-Alpha tocopherol acetate | 0.05 | 0.05 | 0.05 |
| Hydrolyzed collagen *10 | 0.01 | 0.01 | 0.01 |
| Panthenol *11 | 0.05 | 0.05 | 0.05 |
| Panthenyl Ethyl Ether *12 | 0.05 | 0.05 | 0.05 |
| Octyl methoxycinnamate | 0.09 | 0.09 | 0.09 |
| Benzophenone-3 | 0.09 | 0.09 | 0.09 |
| Polyethylene glycol *13 | q.s. to 100% | q.s. to 100% | q.s. to 100% |

| Hair Conditioning Compositions | | | |
|---|---|---|---|
| Components | Ex.4 | Ex. 5 | Ex. 6 |
| Anhydrous magnesium sulfate (MgSO₄) | 35 | - | 20 |
| Anhydrous calcium chloride (CaCl₂) | - | 35 | 20 |
| Polyethylene/polypropylene block copolymer *1 | - | 3.0 | 1.0 |
| PEG modified glyceride *2 | 3.0 | - | 2.0 |
| Cetyl Alcohol *4 | 1.0 | 1.0 | 1.0 |
| Stearyl Alcohol *5 | 1.4 | 1.4 | 1.4 |
| Stearamidopropyl Dimethylamine *6 | 0.8 | 0.8 | 0.8 |
| ℓ-Glutamic acid *7 | 0.256 | 0.256 | 0.256 |
| Hydroxyethylcellulose *8 | 0.5 | 0.5 | 0.5 |
| Perfume | 0.3 | 0.3 | 0.3 |
| Benzyl alcohol | 0.4 | 0.4 | 0.4 |
| EDTA | 0.1 | 0.1 | 0.1 |
| Kathon CG *9 | 0.0005 | 0.0005 | 0.0005 |
| Sodium Chloride | 0.01 | 0.01 | 0.01 |
| 3-pyridinecarboxy acid amide | 0.05 | 0.05 | 0.05 |
| dl-Alpha tocopherol acetate | 0.05 | 0.05 | 0.05 |
| Hydrolyzed collagen *10 | 0.01 | 0.01 | 0.01 |
| Panthenol *11 | 0.05 | 0.05 | 0.05 |
| Panthenyl Ethyl Ether *12 | 0.05 | 0.05 | 0.05 |
| Octyl methoxycinnamate | 0.09 | 0.09 | 0.09 |
| Benzophenone-3 | 0.09 | 0.09 | 0.09 |
| Polyethylene glycol *13 | q.s. to 100% | - | - |
| Glycerin | - | q.s. to 100% | - |
| Pentaerythritol Tetraisostearate *14 | - | - | q.s. to 100% |

| Hair Conditioning Compositions | | | |
|---|---|---|---|
| Components | Ex. 7 | Ex. 8 | Ex. 9 |
| Anhydrous magnesium sulfate (MgSO₄) | 35 | - | - |
| Anhydrous calcium chloride (CaCl₂) | - | 35 | - |
| Anhydrous magnesium chloride (MgCl₂) | - | - | 35 |
| Polyethylene/polypropylene block copolymer *1 | - | - | 3.0 |
| PEG modified glyceride *2 | - | 3.0 | - |
| PEG-60 hydrogenated caster oil *3 | 3.0 | - | |
| Cetyl Alcohol *4 | 1.0 | 1.0 | 1.0 |
| Stearyl Alcohol *5 | 1.4 | 1.4 | 1.4 |
| Stearamidopropyl Dimethylamine *6 | 0.8 | 0.2 | 0.8 |
| ℓ-Glutamic acid *7 | 0.256 | 0.064 | - |
| Hydroxyethylcellulose *8 | 0.1 | 0.1 | 0.1 |
| Silicone blend *15 | 1.0 | 1.0 | 1.0 |
| Polypropylene Glycol *16 | - | 0.5 | - |
| Ditallow dimethyl ammonium chloride *17 | 1.0 | 1.0 | 0.3 |
| PEG-2M *18 | - | - | 0.2 |
| Polysorbate 60 *19 | - | - | 0.2 |
| Cetearyl alcohol *19 | - | - | 0.2 |
| Benzyl alcohol | - | - | 0.2 |
| Glyceryl monostearate *20 | - | - | 0.2 |
| Oleyl alcohol *21 | - | - | 0.2 |
| Perfume | 0.3 | 0.3 | 0.3 |
| Benzyl alcohol | 0.4 | 0.4 | 0.4 |
| EDTA | 0.1 | 0.1 | 0.1 |
| Kathon CG *9 | 0.0005 | 0.0005 | 0.0005 |
| Sodium Chloride | 0.01 | 0.01 | 0.01 |
| 3-pyridinecarboxy acid amide | 0.05 | 0.05 | 0.05 |
| dl-Alpha tocopherol acetate | 0.05 | 0.05 | 0.05 |
| Hydrolyzed collagen *10 | 0.01 | 0.01 | 0.01 |
| Panthenol *11 | 0.05 | 0.05 | 0.05 |
| Panthenyl Ethyl Ether *12 | 0.05 | 0.05 | 0.05 |
| Octyl methoxycinnamate | 0.09 | 0.09 | 0.09 |
| Benzophenone-3 | 0.09 | 0.09 | 0.09 |
| Polyethylene glycol *13 | q.s. to 100% | q.s. to 100% | q.s. to 100% |
| Pentaerythritol Tetraisostearate *14 | - | 1.0 | - |
| Definitions of Components *1 Polyethylene/polypropylene block copolymer: Newpol PE-108 available from Sanyo Chemical. *2 PEG modified glyceride: Tagat TO available from Goldschmidt Chemical Corporation. *3 PEG-60 hydrogenated caster oil : Cremophor RH60 available from BASF. *4 Cetyl Alcohol: Konol series available from Shin Nihon Rika. *5 Stearyl Alcohol: Konol series available from Shin Nihon Rika. *6 Stearamidopropyl Dimethylamine: SAPDMA available from Inolex. *7 ℓ-Glutamic acid: ℓ-Glutamic acid (cosmetic grade) available from Ajinomoto. *8 Hydroxyethylcellulose: Natrosol 250 MBR available from Hercules. *9 Kathon CG: Methylchloroisothiazolinone and Methylisothiazolinone available from Rohm & Haas. *10 Hydrolyzed collagen: Peptein 2000 available from Hormel. *11 Panthenol: available from Roche. *12 Panthenyl Ethyl Ether: available from Roche. *13 Polyethylene glycol: Carbowax PEG-200 available from Union Carbide. *14 Pentaerythritol Tetraisostearate: KAK PTI obtained by Kokyu alcohol. *15 Silicone Blend: SE 76 available from General Electric *16 Polypropylene Glycol: PP2000 available from Sanyo Kasei *17 Ditallow dimethyl ammonium chloride: Available from Witco Chemicals. *18 PEG-2M: Polyox obtained by Union Carbide. *19 Polysorbate 60, Cetearyl Alcohol: mixture sold as Polawax NF obtained by Croda Chemicals. *20 Glycerylmonostearate: Available from Stepan Chemicals. *21 Oleyl alcohol: Available from New Japan Chemical. | | | |

### Method of Preparation

The hair conditioning compositions of Examples 1 through 9 as shown above can be prepared by any conventional method well known in the art. They are suitably made as follows: When included in the composition, polymeric materials such as Hydroxyethylcellulose are dispersed in the inert carrier such as polyethylene glycol (PEG-200) at room temperature to make a polymer solution, and heated up to above 70°C. Phase changing agent such as high melting point fatty compounds, and when present, amidoamines and acids, cationic surfactants, polyoxyalkylene derivatives such as polyoxyethylene/polyoxyalkylene copolymer, and ester oils of low melting point oils are added in the solution with agitation. Then, heat generating agents such as inorganic heat generating agents including magnesium sulfate, calcium chloride, and magnesium chloride are also added in the solution with agitation. The mixture thus obtained is cooled down to about 30°C, and the remaining components such as silicone compound are added with agitation.

### Method of Use

The hair conditioning compositions of Examples 1 through 9 as shown above can be mixed with water and applied to the hair and/or skin by any conventional method well known in the art. For example, the anhydrous compositions can be applied to hair and/or skin after mixing with water on hands and/or in a certain vessel. The anhydrous compositions can be applied to wet hair and/or wet skin to mix with water remaining on the hair and/or skin. The anhydrous compositions can be applied to wet and/or dry hair and/or skin to mix with water when rinsed-off. The hair conditioning compositions of Examples 1 through 9 as shown above are preferably applied to wet hair to mix with water remaining on the hair.

The embodiments disclosed herein have many advantages. For example, anhydrous cosmetic compositions of the present invention, can provide enhanced efficacy, i.e., can provide improved benefits, while reducing gritty feel to the skin and/or hair. For example, the hair conditioning compositions can provide improved hair conditioning benefits such as moisturized feel, softness, and static control to the hair, due to improved penetration of ingredients, while preventing the compositions from warming up to a higher temperature than expected, and can also provide prolonged warming from the compositions.

## Claims

1. An anhydrous hair care and/or skin care cosmetic composition comprising:
(a) a heat generating agent which generates a heat by mixing with water;
(b) a phase changing agent; and
(c) an inert carrier;
wherein the phase changing agent has a melting point of from 30°C to 70°C and is dispersed in the inert carrier and
wherein the heat generating agent is an anhydrous inorganic salt selected from the group consisting of sodium sulfate, calcium sulfate, magnesium sulfate, aluminum sulfate, calcium chloride, magnesium chloride, calcium oxide, and mixtures thereof.

2. The anhydrous cosmetic composition according to Claim 1, wherein the phase changing agent is selected from the group consisting of amidoamines, fatty alcohols, fatty adds, fatty alcohol derivatives, fatty acid derivatives, and mixtures thereof.

3. The anhydrous cosmetic composition according to Claims 1 and 2, wherein the phase changing agent is selected from the group consisting of cetyl alcohol, stearyl alcohol, and mixtures thereof.

4. The anhydrous cosmetic composition according to any of the preceding Claims, wherein the inert carrier is selected from the group consisting of polyethylene glycol, polypropylene glycol, glycerin, liquid paraffin, mineral oil, vegetable oil, penteerythritol tetraisostearate, and mixtures thereof.

5. The anhydrous cosmetic composition according to Claim 4, wherein the inert carrier is polyethylene glycol.

6. The anhydrous cosmetic composition according to Claim 1, wherein the inert carrier is polyethylene glycol and the phase changing agent is selected from the group consisting of cetyl alcohol, stearyl alcohol, and mixtures thereof.

7. The anhydrous cosmetic composition according to any of the preceding Claims further comprising a polyoxyalkylene derivative selected from the group consisting of polyoxyethylene/polyoxypropylene copolymer, polyoxyethylene alkyl ether, polyoxypropylene alkyl ether, polyoxyethylene alkyl ether ester, polyoxypropylene alkyl ether ester, polyoxyethylene glyceryl ester, polyoxypropylene glyceryl ester, and mixtures thereof

8. The anhydrous cosmetic composition according to Claim 7, wherein the polyoxyalkylene derivative is polyoxyethylene/polyoxypropylene copolymer.

9. The anhydrous cosmetic composition according to Claim 7, wherein the polyoxyalkylene derivative is polyoxyethylene/polyoxypropylene block copolymer.

10. The anhydrous cosmetic composition according to any of the preceding Claims further comprising a reaction control agent selected from the group consisting of cellulose derivatives, modified cellulose polymers, and mixtures thereof.

11. The anhydrous cosmetic composition according to any of the preceding Claims, which warms to a temperature of from about 30°C to about 80°C by mixing with water.

12. The anhydrous cosmetic composition according to any of the preceding Claims, which is an anhydrous hair care composition selected from the group consisting of an anhydrous hair shampoo composition, an anhydrous hair styling composition, an anhydrous hair conditioning composition, an anhydrous hair colour composition, an anhydrous hair growth composition, and mixtures thereof.

13. The anhydrous cosmetic composition according to Claim 12, which is an anhydrous hair conditioning composition.

14. The anhydrous cosmetic composition according to Claim 13, wherein the anhydrous hair conditioning composition further comprises a high melting point fatty compound.

15. The anhydrous cosmetic composition according to Claim 12, wherein the anhydrous hair conditioning composition further comprises an amidoamine having the following general formula:
R¹ CO NH (CH₂)ₘ N (R²)₂
wherein R¹ is a residue of C11 to C24 fatty acids, R² is a C 1 to C4 alkyl, and m is an integer from 1 to 4.

16. The anhydrous cosmetic composition according to Claim 15, wherein the anhydrous hair conditioning composition further comprises an add selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof, at a level such that the mole ratio of amidoamine to acid is from about 1:0.3 to about 1:1.

17. The anhydrous cosmetic composition according to Claim 12, wherein the anhydrous hair conditioning composition comprises by weight:
(a) from about 5% to about 60% of the heat generating agent which generates a heat by mixing with water;
(b) from about 0.1% to about 30% of the phase changing agent selected from the group consisting of fatty alcohols, fatty acids, fatty alcohol derivatives, fatty acid derivatives, and mixtures thereof;
(c) from about 0.1 % to about 10% of a polyoxyalkylene derivative selected from the group consisting of polyoxyethylene/polyoxypropylene copolymer, polyoxyethylene alkyl ether, polyoxypropylene alkyl ether, polyoxyethylene alkyl ether ester, polyoxypropylene alkyl ether ester, polyoxyethylene glyceryl ester, polyoxypropylene glyceryl ester, and mixtures thereof;
(d) from about 0.05% to about 10% of an amidoamine having the following general formula:
R¹ CO NH (CH₂)ₘ N (R²)₂
wherein R¹ is a residue of C 11 to C24 fatty acids, R² is a C 1 to C4 alkyl, and m is an integer from 1 to 4;
(e) an acid selected from the group consisting of e-glutamic acid, lactic acid, hydrochloric acid, malic acid, succinic acid, acetic acid, fumaric acid, ℓ-glutamic acid hydrochloride, tartaric acid, and mixtures thereof, at a level such that the mole ratio of amidoamine to acid is from about 1:0.3 to about 1:1: and
(f) an inert carrier.

18. A method of using the hair conditioning composition according to Claim 13, wherein the composition is applied to wet hair to mix with water remaining on the hair.

## Patentansprüche

1. Wasserfreie Haarpflege- und Hautpflegekosmetikzusammensetzung, umfassend:
(a) ein wärmeerzeugendes Mittel, welches durch Mischen mit Wasser Wärme erzeugt;
(b) ein phasenänderndes Mittel; und
(c) einen inerten Träger;
wobei das phasenändernde Mittel einen Schmelzpunkt von 30 °C bis 70 °C aufweist und in dem inerten Träger dispergiert ist und wobei das wärmeerzeugende Mittel ein wasserfreies anorganisches Salz ist, ausgewählt aus der Gruppe bestehend aus Natriumsulfat, Calciumsulfat, Magnesiumsulfat, Aluminiumsulfat, Calciumchlorid, Magnesiumchlorid, Calciumoxid und Mischungen davon.

2. Wasserfreie kosmetische Zusammensetzung nach Anspruch 1, wobei das phasenändernde Mittel ausgewählt ist aus der Gruppe bestehend aus Amidoaminen, Fettalkoholen, Fettsäuren, Fettalkoholderivaten, Fettsäurederivaten und Mischungen davon.

3. Wasserfreie kosmetische Zusammensetzung nach Anspruch 1 und 2, wobei das phasenändernde Mittel ausgewählt ist aus der Gruppe bestehend aus Cetylalkohol, Stearylalkohol und Mischungen davon.

4. Wasserfreie kosmetische Zusammensetzung nach einem der vorstehenden Ansprüche, worin der inerte Träger ausgewählt ist aus der Gruppe bestehend aus Polyethylenglycol, Polypropylenglycol, Glycerin, flüssigem Paraffin, Mineralöl, Pflanzenöl, Pentaerythrittetraisostearat und Mischungen davon.

5. Wasserfreie kosmetische Zusammensetzung nach Anspruch 4, wobei der inerte Träger Polyethylenglycol ist.

6. Wasserfreie kosmetische Zusammensetzung nach Anspruch 1, wobei der inerte Träger Polyethylenglycol ist und das phasenändernde Mittel ausgewählt ist aus der Gruppe bestehend aus Cetylalkohol, Stearylalkohol und Mischungen davon.

7. Wasserfreie kosmetische Zusammensetzung nach einem der vorstehenden Ansprüche, die ferner ein Polyoxyalkylenderivat, ausgewählt aus der Gruppe bestehend aus Polyoxyethylen/Polyoxypropylen-Copolymer, Polyoxyethylenalkylether, Polyoxypropylenalkylether, Polyoxyethylenalkyletherester, Polyoxypropylenalkyletherester, Polyoxyethylenglycerylester, Polyoxypropylenglycerylester und Mischungen davon, umfasst.

8. Wasserfreie kosmetische Zusammensetzung nach Anspruch 7, wobei das Polyoxyalkylenderivat Polyoxyethylen/Polyoxypropylen-Copolymer ist.

9. Wasserfreie kosmetische Zusammensetzung nach Anspruch 7, wobei das Polyoxyalkylenderivat Polyoxyethylen/Polyoxypropylen-Blockcopolymer ist.

10. Wasserfreie kosmetische Zusammensetzung nach einem der vorstehenden Ansprüche, die ferner ein Reaktionslenkungsmittel, ausgewählt aus der Gruppe bestehend aus Cellulosederivaten, modifizierten Cellulosepolymeren und Mischungen davon, umfasst.

11. Wasserfreie kosmetische Zusammensetzung nach einem der vorstehenden Ansprüche, die sich durch Mischen mit Wasser auf eine Temperatur von etwa 30 °C bis etwa 80 °C erwärmt.

12. Wasserfreie kosmetische Zusammensetzung nach einem der vorstehenden Ansprüche, die eine wasserfreie Haarpflegezusammensetzung ist, ausgewählt aus der Gruppe bestehend aus einer wasserfreien Haarshampoozusammensetzung, einer wasserfreien Haarstylingzusammensetzung, einer wasserfreien Haarkonditionierungszusammensetzung, einer wasserfreien Haarfärbezusammensetzung, einer wasserfreien Haarwuchszusammensetzung, und Mischungen davon.

13. Wasserfreie kosmetische Zusammensetzung nach Anspruch 12, die eine wasserfreie Haarkonditionierungszusammensetzung ist.

14. Wasserfreie kosmetische Zusammensetzung nach Anspruch 13, wobei die wasserfreie Haarkonditionierungszusammensetzung ferner eine Fettverbindung mit hohem Schmelzpunkt umfasst.

15. Wasserfreie kosmetische Zusammensetzung nach Anspruch 12, wobei die wasserfreie Haarkonditionierungszusammensetzung ferner ein Amidoamin mit folgender allgemeiner Formel umfasst:
R¹CONH(CH₂)ₘN(R²)₂
worin R¹ ein Rest von C11- bis C24-Fettsäuren ist, R² ein C1- bis C4-Alkyl ist und m eine ganze Zahl von 1 bis 4 ist.

16. Wasserfreie Kosmetikzusammensetzung nach Anspruch 15, wobei die wasserfreie Haarkonditionierungszusammensetzung ferner eine Säure, ausgewählt aus der Gruppe bestehend aus ℓ-Glutaminsäure, Milchsäure, Salzsäure, Äpfelsäure, Bernsteinsäure, Essigsäure, Fumarsäure, ℓ-Glutaminsäurehydrochlorid, Weinsäure und Mischungen davon, in einer solchen Konzentration, dass das Molverhältnis von Amidoamin zu Säure von ungefähr 1:0,3 bis ungefähr 1:1 beträgt, umfasst.

17. Wasserfreie kosmetische Zusammensetzung nach Anspruch 12, wobei die wasserfreie Haarkonditionierungszusammensetzung Folgendes in Gewichtsprozent umfasst:
(a) zu etwa 5 % bis etwa 60 % das wärmeerzeugende Mittel, das Wärme durch Mischung mit Wasser erzeugt;
(b) zu etwa 0,1 % bis etwa 30 % das phasenändernde Mittel, ausgewählt aus der Gruppe bestehend aus Fettalkoholen, Fettsäuren, Fettalkoholderivaten, Fettsäurederivaten und Mischungen davon;
(c) zu etwa 0,1 % bis etwa 10 % ein Polyoxyalkylenderivat, ausgewählt aus der Gruppe bestehend aus Polyoxyethylen/Polyoxypropylen-Copolymer, Polyoxyethylenalkylether, Polyoxypropylenalkylether, Polyoxyethylenalkyletherester, Polyoxypropylenalkyletherester, Polyoxyethylenglycerylester, Polyoxypropylenglycerylester und Mischungen davon;
(d) zu etwa 0,05 % bis etwa 10 % ein Amidoamin mit folgender allgemeiner Formel:
R¹CONH(CH₂)ₘN(R²)₂
worin R¹ ein Rest von C11- bis C24-Fettsäuren ist, R² ein C1- bis C4-Alkyl ist und m eine ganze Zahl von 1 bis 4 ist;
(e) eine Säure, ausgewählt aus der Gruppe, bestehend aus ℓ-Glutaminsäure, Milchsäure, Salzsäure, Äpfelsäure, Bernsteinsäure, Essigsäure, Fumarsäure, ℓ-Glutaminsäurehydrochlorid, Weinsäure und Mischungen davon, in einer solchen Konzentration, dass das Molverhältnis von Amidoamin zu Säure von ungefähr 1:0,3 bis ungefähr 1:1 beträgt; und
(f) einen inerten Träger.

18. Verfahren zur Verwendung der Haarkonditionierungszusammensetzung nach Anspruch 13, wobei die Zusammensetzung auf das feuchte Haar aufgetragen wird, um sich mit dem Wasser zu mischen, das auf dem Haar verblieben ist.

## Revendications

1. Composition cosmétique anhydre pour le soin capillaire et /ou le soin de la peau comprenant :
(a) un agent de génération de chaleur qui génère de la chaleur lorsqu'il est mélangé à de l'eau ;
(b) un agent de changement de phase ; et
(c) un véhicule inerte ;
dans laquelle l'agent de changement de phase a un point de fusion allant de 30 °C à 70 °C et est dispersé dans le véhicule inerte et dans laquelle l'agent de génération de chaleur est un sel inorganique anhydre choisi parmi le groupe constitué de sulfate de sodium, sulfate de calcium, sulfate de magnésium, sulfate d'aluminium, chlorure de calcium, chlorure de magnésium, et oxyde de calcium, et leurs mélanges.

2. Composition cosmétique anhydre selon la revendication 1, dans laquelle l'agent de changement de phase est choisi dans le groupe constitué d'amidoamines, alcools gras, acides gras, dérivés d'alcool gras, dérivés d'acide gras, et leurs mélanges.

3. Composition cosmétique anhydre selon les revendications 1 et 2, dans laquelle l'agent de changement de phase est choisi dans le groupe constitué d'alcool cétylique, alcool stéarylique, et leurs mélanges.

4. Composition cosmétique anhydre selon l'une quelconque des revendications précédentes, dans laquelle le véhicule inerte est choisi dans le groupe constitué de polyéthylène glycol, polypropylène glycol, glycérine, paraffine liquide, huile minérale, huile végétale, tétra-isostéarate de penta-érythritol, et leurs mélanges.

5. Composition cosmétique anhydre selon la revendication 4, dans laquelle le véhicule inerte est du polyéthylène glycol.

6. Composition cosmétique anhydre selon la revendication 1, dans laquelle le véhicule inerte est du polyéthylène glycol, et l'agent de changement de phase est choisi dans le groupe constitué d'alcool cétylique, alcool stéarylique, et leurs mélanges.

7. Composition cosmétique anhydre selon l'une quelconque des revendications précédentes, comprenant en outre un dérivé de polyoxyalkylène choisi dans le groupe constitué d'un copolymère polyoxyéthylène/polyoxypropylène, un polyoxyéthylène alkyléther, une polyoxypropylène alkyléther, un ester de polyoxyéthylène alkyléther, un ester de polyoxypropylène alkyléther, un ester de polyoxyéthylène glycéryle, un ester de polyoxypropylène glycéryle, et leurs mélanges.

8. Composition cosmétique anhydre selon la revendication 7, dans laquelle le dérivé de polyoxyalkylène est un copolymère polyoxyéthylène/polyoxypropylène.

9. Composition cosmétique anhydre selon la revendication 7, dans laquelle le dérivé de polyoxyalkylène est un copolymère séquencé polyoxyéthylène/polyoxypropylène.

10. Composition cosmétique anhydre selon l'une quelconque des revendications précédentes, comprenant en outre un agent de contrôle de réaction choisi dans le groupe constitué de dérivés de cellulose, polymères de cellulose modifiés, et leurs mélanges.

11. Composition cosmétique anhydre selon l'une quelconque des revendications précédentes, qui s'échauffe à une température allant d'environ 30 °C à environ 80 °C par mélange avec de l'eau.

12. Composition cosmétique anhydre selon l'une quelconque des revendications précédentes, qui est une composition pour soins capillaires anhydre choisie parmi le groupe constitué d'une composition de shampoing pour cheveux anhydre, une composition de coiffage anhydre, une composition de conditionnement des cheveux anhydre, une composition de colorant pour cheveux anhydre, une composition de pousse des cheveux anhydre, et leurs mélanges.

13. Composition cosmétique anhydre selon la revendication 12, qui est une composition de conditionnement des cheveux anhydre.

14. Composition cosmétique anhydre selon la revendication 13, dans laquelle la composition de conditionnement des cheveux anhydre comprend en outre un composé gras à point de fusion élevé.

15. Composition cosmétique anhydre selon la revendication 12, dans laquelle la composition de conditionnement des cheveux anhydre comprend en outre une amidoamine de formule générale suivante :
R¹CONH(CH₂)ₘN(R²)₂
dans laquelle R¹ est un résidu d'acides gras en C11 à C24, R² est un alkyle en C1 à C4, et m est un nombre entier allant de 1 à 4.

16. Composition de produit de beauté anhydre selon la revendication 15, dans laquelle la composition de conditionnement des cheveux anhydre comprend en outre un acide choisi parmi le groupe constitué d'acide L-glutamique, acide lactique, acide chlorhydrique, acide malique, acide succinique, acide acétique, acide fumarique, chlorhydrate d'acide L-glutamique, acide diacétyltartrique, et leurs mélanges, à un taux tel que le rapport molaire de l'amidoamine sur l'acide est d'environ 1:0,3 à environ 1:1.

17. Composition cosmétique anhydre selon la revendication 12, dans laquelle la composition de conditionnement des cheveux anhydre comprend en poids :
(a) d'environ 5 % à environ 60 % de l'agent de génération de chaleur qui génère de la chaleur par mélange avec de l'eau ;
(b) d'environ 0,1 % à environ 30 % de l'agent de changement de phase choisi dans le groupe constitué d'alcools gras, acides gras, dérivés d'alcool gras, dérivés d'acide gras, et leurs mélanges ;
(c) d'environ 0,1 % à environ 10 % d'un dérivé de polyoxyalkylène choisi dans le groupe constitué d'un copolymère polyoxyéthylène/polyoxypropylène, un polyoxyéthylène alkyléther, un polyoxypropylène alkyléther, un ester de polyoxyéthylène alkyléther, un ester de polyoxypropylène alkyléther, un ester de polyoxyéthylène glycéryle, un ester de polyoxypropylène glycéryle, et leurs mélanges ;
(d) d'environ 0,05 % à environ 10 % d'une amidoamine de formule générale suivante :
R¹CONH(CH₂)ₘN(R²)₂
dans laquelle R¹ est un résidu d'acides gras en C11 à C24, R² est un alkyle en C11 à C4, et m est un nombre entier allant de 1 à 4 ;
(e) un acide choisi parmi le groupe constitué d'acide L-glutamique, acide lactique, acide chlorhydrique, acide malique, acide succinique, acide acétique, acide fumarique, chlorhydrate d'acide L-glutamique, acide diacétyltartrique, et leurs mélanges, à un niveau tel que le rapport molaire de l'amidoamine sur l'acide est d'environ 1:0,3 à environ 1:1 ; et
(f) un véhicule inerte.

18. Procédé d'utilisation d'une composition de conditionnement des cheveux selon la revendication 13, dans lequel la composition est appliquée sur cheveux humides pour se mélanger avec l'eau restant sur les cheveux.
